# EUROPEAN PATENT SPECIFICATION

(11) **EP 3 186 391 B1**
(45) Date of publication and mention of the grant of the patent: **27.05.2020**
(21) Application number: 15763832.1
(22) Date of filing: 28.08.2015
(51) Int. Cl.: C12Q 1/68

(54) **METHODS AND KIT FOR IDENTIFICATION OF CANCER**
VERFAHREN UND KIT ZUR FESTSTELLUNG VON ENTZÜNDETEM UND MALIGNEM INTESTINALGEWEBE
PROCÉDÉ ET KIT POUR L'IDENTIFICATION DE TUMEUR MALINE ET INFLAMMATION DU TISSU INTESTINAL

(30) Priority: 28.08.2014 EP 14182681; 27.03.2015 GB 201505294
(43) Date of publication of application: 05.07.2017
(73) Proprietor: The Provost, Fellows, Foundation Scholars, & the other members of Board, of the College of Holy and Undiv. Trinity of Queen Elizabeth near Dublin, 2 Dublin (IE)
(72) Inventor: CREAGH, Emma, Dublin 8 (IE); FLOOD, Brian, Drogheda County Meath (IE); KAY, Elaine, Dublin 14 (IE); OFICJALSKA, Katarzyna, Dublin 8 (IE); RYAN, Elizabeth, Portmarnock County Dublin (IE); DOHERTY, Glen, Dublin 8 (IE); SALTO-TELLEZ, Manual, Belfast BT9 7BL (GB)
(74) Representative: FRKelly
(86) International application number: PCT/EP2015/001753
(87) International publication number: WO 2016/030020

(56) References cited:
- WO-A1-2012/158385
- WO-A2-2007/013665
- WO-A2-2009/073565
- WO-A2-2009/120877
- US-A1- 2008 014 579
- PHILCHENKOV ALEKSEI ET AL: "Caspases and cancer: mechanisms of inactivation and new treatment modalities.", EXPERIMENTAL ONCOLOGY JUN 2004, vol. 26, no. 2, June 2004 (2004-06), pages 82-97, XP055228742, ISSN: 1812-9269
- KOBAYASHI TAIRA ET AL: "TheShigellaOspC3 Effector Inhibits Caspase-4, Antagonizes Inflammatory Cell Death, and Promotes Epithelial Infection", CELL HOST & MICROBE, ELSEVIER, NL, vol. 13, no. 5, 15 May 2013 (2013-05-15), pages 570-583, XP028564664, ISSN: 1931-3128, DOI: 10.1016/J.CHOM.2013.04.012
- CHRISTOPH BECKER ET AL: "Complex Roles of Caspases in the Pathogenesis of Inflammatory Bowel Disease", GASTROENTEROLOGY, vol. 144, no. 2, 1 February 2013 (2013-02-01), pages 283-293, XP055168296, ISSN: 0016-5085, DOI: 10.1053/j.gastro.2012.11.035
- JEREMY DUPAUL-CHICOINE ET AL: "Control of Intestinal Homeostasis, Colitis, and Colitis-Associated Colorectal Cancer by the Inflammatory Caspases", IMMUNITY, vol. 32, no. 3, 1 March 2010 (2010-03-01), pages 367-378, XP055168332, ISSN: 1074-7613, DOI: 10.1016/j.immuni.2010.02.012

## Description

This invention relates to in vitro methods for the diagnosis, identification and/or staging of GI tract carcinoma or adenocarcinoma, including the diagnosis, identification and/or staging of inflamed or malignant tissue and/or associated disease state. The invention also relates to caspases or caspase-modulators for use in the treatment of carcinoma in a subject.

### BACKGROUND

### Cancer

Cancer develops from an outgrowth of a single population of cells from a tissue in the body. Carcinomas are cancers that begin in epithelial tissues. Epithelial tissue is specialised to form the covering or lining of all internal and external body surfaces, and is made up of cells closely packed and arranged into one or more layers. Epithelial tissue that occurs on surfaces on the interior of the body is known as endothelium. Epithelial tissues include the skin, which covers the body, and other tissues that line or cover internal organs, such as those of the digestive system. There are a number of different epithelial cell types, which give rise to a number of carcinoma subtypes, including: adenocarcinomas, which originate from glandular adenomatous cells (such as those lining the colon and rectum); squamous cell carcinomas, which originate from squamous epithelial cells (such as those lining the throat and oesophagus); transitional cell carcinoma, which originate from transitional epithelial cells, which can expand with the organ (such as those lining the bladder); and basal cell carcinoma; which originate from the deepest layer of skin cells and are uncommon.

Adenocarcinomas are the most common type of carcinoma, and are responsible for the majority of cancers associated with the Colon, Gastro-Intestinal tract, Breast, Vagina, Prostate and Throat. Approximately 40% of lung cancers are adenocarcinoma, with squamous cell carcinoma, small cell carcinoma and large cell carcinoma being responsible for the remaining 60%.

Gastro-intestinal (GI) malignancy is a term for a group of cancers, primarily adenocarcinomas, that affect the digestive system. GI cancer is the most common form of cancer, and includes cancers of the oesophagus, stomach, biliary tract, pancreas, small intestine, large intestine (colon), rectum and anus. Most GI cancers are carcinomas, such as those commonly referred to as stomach cancer, colon cancer etc. The majority of GI cancers are detected during endoscopic procedures, during which tissue biopsies are taken and pathologically assessed to confirm and stage the malignancy. In the case of pancreatic and biliary tract cancers, imaging techniques are usually employed to identify malignant tissue, however subsequent endoscopic ultrasound (EUS) procedures, including tissue sampling using fine needle aspiration, are performed to stage the malignancy. Accurate staging of invasive cancers is essential to determine prognosis and treatment, thus extensive tumour sampling is generally required.

Breast cancers are a collective term given to any cancers that form in tissues of the breast. As with GI cancers, the majority of breast cancers are carcinomas (i.e. the cancer is derived from epithelial tissue). The most common type of breast cancer is ductal carcinoma, which begins in the epithelial lining of the milk ducts (thin tubes that carry milk from the lobules of the breast to the nipple). Another type of breast cancer is lobular carcinoma, which begins in the epithelial layer of the breast lobules (milk glands). Invasive breast cancer is breast cancer that has spread from where it began in the breast ducts or lobules to surrounding normal tissue. Breast cancer occurs in both men and women, although male breast cancer is rare.

The causes of carcinomas are multi-factorial, which can include genetics and environmental factors, such as lifestyle and diet. Risk factors vary according to which tissue the carcinoma is associated, for example inherited mutations in the BRCA1 or BRCA2 genes increase risk of breast and/or ovarian cancer; and tobacco smoking increases risk of lung, throat and oesophageal adenocarcinoma.

The development of cancer is reliant on seven fundamental features of the cancer cell/tumour: 1. ability to evade cell death (apoptosis); 2. enabling replicative immortality; 3. sustaining proliferative signalling; 4. evading growth suppressors; 5. inducing angiogenesis; 6. activating invasion and metastasis; and 7. an inflammatory microenvironment. Each of these features is being intensely researched to enhance understanding of cancer development mechanisms and to develop strategies to halt cancer progression. The final feature listed above, the presence of an inflammatory microenvironment, has only been recently added to the list. It is also clear that persistent, sustained inflammation within cellular tissue can predispose the inflamed area to the development of malignancy. For example, in the oesophagus, long standing inflammation caused by acid reflux causes squamous cells to be replaced by columnar epithelial cells (known as Barrett's oesophagus). Patients with Barrett's oesophagus are at increased risk of developing oesophageal adenocarcinoma (OAC). Patients with a history of persistent inflammation of the stomach lining, known as gastritis, are at increased risk of developing gastric cancer. Patients with gallbladder inflammation, caused by infection or gallstones, are at increased risk of developing gallbladder cancer, and patients with primary sclerosing cholangitis (PSC) have inflamed bile ducts and increased risk of cancer of the gallbladder and bile ducts. Of note, many PSC patients also have Ulcerative Colitis (UC), this disease is known as PSC-IBD, and these patients are therefore have an even greater risk for developing a malignancy within either the colon or gallbladder. Furthermore, chronic or hereditary pancreatitis is a risk factor for pancreatic cancer.

The present invention relates to in vitro methods for diagnosis of inflamed or malignant tissue. In addition, the new methods can also assist in the diagnosis of carcinomas, preferably adenocarcinomas, of the colon, and GI tract. Preferably, the method can also assist in the early stage diagnosis of adenocarcinomas, of the colon, and GI tract.

### Inflammatory Bowel Disease (IBD) and Colerectal Cancer (CRC)

As previously discussed, there is a increased risk of cancer within severely inflamed tissues, and the pathogenesis of 15% of all human cancers have now been linked to inflammation. One of the most prevalent inflammation driven cancers is colitis-associated colorectal cancer. Colorectal cancer (CRC) is the third most common cancer in the World. In Ireland, CRC is responsible for 15% of all cancer related deaths with an average of 1445 cases (both men and women) being diagnosed each year (National Cancer Registry Ireland). The cancer can develop either spontaneously (neoplastic) or as a result of chronic inflammation (dysplastic).

Colorectal tumours are predominantly derived from epithelial cells and present with a broad spectrum of neoplasms ranging from benign growths to invasive metastases. About 95% of CRCs are adenocarcinomas, which are defined as neoplasias of epithelial tissue with glandular characteristics, origin or both. Most adenocarcinomas arise from adenomatous polyps (adenomas). It is well recognized that adenomatous polyps are benign tumours that may (or may not) undergo malignant transformation. It is widely accepted that the removal of adenomatous polyps is associated with a reduced risk of CRC.

The adenoma-carcinoma progression seen in sporadic CRC is not seen in IBD. In IBD, particularly in UC, the progression is suggested to proceed from inflammation to dysplasia and then to cancer - the dysplasia-carcinoma sequence. There are, however, exceptions to this paradigm, in which patients will be observed to develop CRC without having had dysplasia.

IBD is an example of a chronic inflammatory condition that can greatly increase the threat of CRC. Inflammatory bowel disease (IBD) is characterized by chronic, recurrent inflammation of the gastrointestinal tract and has two main forms - Crohn's disease (CD) and Ulcerative colitis (UC). People with IBD (ulcerative colitis and Crohn's disease) are at increased risk of colon cancer. To date, although intestinal inflammation is a feature inflammatory bowel disease (IBD) and colorectal cancer (CRC), the mechanisms driving inflammation are still unclear.

The prevalence of IBD in Western countries is estimated at 1/1,000 inhabitants, with worldwide prevalence increasing over the last 50 years. Numerous studies have consistently supported the oncogenic impact of chronic inflammation on colorectal mucosa, thus IBD patients have an associated risk of developing colorectal cancer (CRC), with cumulative 30 year risk of UC-associated CRC of close to 20%. CRC represents the second most common incident solid organ cancer in females (after breast cancer) and males (after prostate cancer), with an estimated annual incidence in 2008 of 1.2 million worldwide. The pathogenesis of CRC is complex, and is influenced by genetics, lifestyle and dietary factors, however an inflammatory microenvironment is a critical component for CRC progression, regardless of the initiation pathway.

Currently, pathologists analyse biopsy/resection tissue using standard H&E staining techniques to observe the tissue histology and characterise the tissue as being normal/inflamed/dysplastic/neoplastic, in addition to the size, frequency and type of adenomas. Moderately differentiated adenocarcinomas are usually present in colorectal cancers, and these are usually staged using the Dukes' system (stage A-confined to bowel muscle wall, stage B- invasion through muscle wall and stage C- presence of lymph node metastases) and the TNM (Tumour-Node-Metastasis) system. The pathologist guidelines for assigning tumour (T) grades are: T1, tumour is confined to the inner layer of the bowel; T2, tumour has advanced into the muscle layer of the bowel wall; T3, tumour has grown into the outer lining of the bowel wall; and T4, tumour has invaded the bowel wall with ulceration and/or invasion into adjacent organs. The TNM system has the advantage of subdividing the Dukes' stage B tumours into T3 and T4.

Once established, colorectal carcinoma is readily detectable by pathologists. However, the early stages of progression to CRC, particularly distinguishing between inflammation and true dysplasia, is not as straightforward, and there is a large amount of variation between pathologists with regard to the identification and grading of dysplasia. Patients who have had colitis for >10 years are advised to undergo regular colonoscopy to detect preinvasive neoplastic lesions. Detection of high grade dysplasia is also regarded as an indication for colectomy. However, colectomy is a serious procedure for the patient, and has a major impact on their quality of life, therefore this decision is not made lightly. Pathologists often agonise over whether resection tissue from a patient is inflamed, which will resolve, or has become truly dysplastic, in which case the best option is colectomy.

Thus, there are no defined clinical features to predict progression from inflammation to low grade dysplasia, and on to advanced neoplasia, and histological assessment of biopsy tissue is heavily relied upon when making decisions regarding the management of IBD and the associated risk of CRC development. Therefore, there is a serious requirement for novel identifiers of dysplasia and neoplasia, which can be pathologically assessed in parallel with the current system of tissue histology assessment, for the identification and diagnosis of CRC.

WO 2012158385 discloses a method for detecting MSH6-deficient colorectal cancers (CRCs) in a human subject comprising the steps of: identifying the human subject suspected of having the MSH6-deficient CRC; obtaining a tissue sample from the human subject; isolating a DNA from the tissue sample; amplifying the DNA in a PCR system comprising: a CAT25 mononucleotide marker; and at least one other marker selected from the group consisting of BAT25, BAT26, NR21, NR22, NR24, NR27; detecting the presence of the MSH6-deficient CRC based on a reduced expression or an inactivation of the MSH6 gene, MSH6 protein on, or both in the tissue sample of the human subject suspected of having the MSH6-deficient CRC.

WO 2009120877 discloses a method of determining whether a subject is afflicted with an inflammatory bowel disease, condition, or subtype thereof, the method comprising: a) determining the level of expression or activity of a biomarker listed in Tables 2-14 or a fragment thereof in a subject sample; b) determining the normal level of expression or activity of the biomarker in a control sample; and c) comparing the level of expression or activity of said biomarker detected in steps a) and b); wherein a significant modulation in the level of expression or activity of the biomarker in the subject sample relative to the normal level of expression or activity of the biomarker in a control sample is an indication that the subject is afflicted with an inflammatory bowel disease, condition, or a subtype thereof.

Caspases are a group of proteolytic enzymes involved in the co-ordination of cellular processes, including cellular homeostasis, inflammation and cell death. In humans, caspases-1, -4 and -5 constitute the inflammatory caspase subfamily. In humans, the CASP1, CASP5 and CASP4 genes form a cluster on chromosome 11. Caspase-1 shares 53% identity to caspase-4 and 51% identity to caspase-5. Caspase-1 has two known substrates, IL-1beta and IL-18, both of which are potent pro-inflammatory mediators. However, no substrates for caspases-4 or -5 have been identified to date. Although they exhibit significant amino acid identity to caspase-1, caspases-4 and -5 have been shown to have different substrate affinities and specificities to caspase-1. For example, caspase-4 and -5 cannot cleave IL-1beta or IL-18 at physiological levels. Furthermore, a peptide inhibitor of caspase-1 does not inhibit the activity of caspase-4 or -5 [1]. Excessive production of the inflammatory cytokines IL-1β and IL-18 is found in inflamed colons of IBD patients [2, 3]. IL-1β expression is also upregulated during CRC and has been identified as a key factor in tumor progression and metastasis [4].

Although caspase-4 activation has been previously linked to gastric ulcer formation, it has not been previously associated with gastric cancer [5]. A number of mutational analysis studies have been carried out to determine whether alterations in inflammatory caspase genes may be involved in the development of human cancers [6]. These have revealed that the overall incidence of solid tumor mutations are rare for caspase-1 and -4, and occasional for caspase-5 [6, 7], suggesting that mutations in inflammatory caspases are not causally implicated in oncogenesis.

The revelation that murine caspase-11 is involved in regulating IL-1β production following Gram negative bacterial infection [8] has led us to investigate whether human caspases-4 and -5, which have been proposed as being functional homologs of murine caspase-11, may be involved in mediating intestinal inflammation during IBD and CRC. However, caspase-11 knockout mice have recently been reported to exhibit excessive intestinal inflammation in an experimental model of colitis, suggesting that caspase-11 negatively regulates intestinal inflammation [9].

The present invention relates to an in vitro method for diagnosis of inflamed or malignant tissue, and advantageously associated and GI tract malignancy. In addition, the method can also assist in the diagnosis of GI tract malignancy, preferably early stage and GI tract malignancy.

Specifically, the present invention relates to in vitro methods for diagnosis of inflamed or malignant intestinal tissue associated with Inflammatory bowel disease (IBD), Crohn's disease (CD), Ulcerative Colitis (UC) and Colorectal Cancer (CRC). In addition, new methods can also assist in the diagnosis of IBD and the diagnosis of dysplasia and neoplasia from intestinal samples.

### STATEMENTS OF THE INVENTION

Unless otherwise defined, all terms of art, notations and other scientific terminology used herein are intended to have the meanings commonly understood by those skilled in the art to which this disclosure pertains. In some cases, terms with commonly understood meanings are defined herein for clarity and/or for ready reference; thus, the inclusion of such definitions herein should not be construed to represent a substantial difference over what is generally understood in the art.

The term "approximately" herein refers to the range of the experimental error, which may occur in a measurement.

The terms "comprising", "having", "including" and "containing" are to be construed as open-ended terms (i.e. meaning "including, but not limited to") and are to be considered as including and.or embracing the terms as "consist essentially of", "consisting essentially of", "consist of" or "consisting of".

As used herein, the terms "therapeutically effective amount" and "effective amount" refer to an amount sufficient to elicit the desired biological response. In the present invention the desired biological response is to inhibit, reduce or ameliorate the severity, duration, progression, or onset of a disease, disorder or condition, prevent the advancement, recurrence, or progression of a disease, disorder or condition or a symptom associated with a disease, disorder or condition. The precise amount of compound administered to a subject will depend on the mode of administration, the type and severity of the disease, disorder or condition and on the characteristics of the subject, such as general health, age, sex, body weight and tolerance to drugs. The skilled artisan will be able to determine appropriate dosages depending on these and other factors.

As used herein, the terms "treat", "treatment" and "treating" refer to therapeutic treatments includes the reduction or amelioration of the progression, severity and/or duration of a disease, disorder or condition, or the amelioration of one or more symptoms (specifically, one or more discernible symptoms) of a disease, disorder or condition, resulting from the administration of one or more therapies (e.g., one or more therapeutic agents such as a compound or composition of the invention). In specific embodiments, the therapeutic treatment includes the amelioration of at least one measurable physical parameter of a disease, disorder or condition. In other embodiments the therapeutic treatment includes the inhibition of the progression of a condition, either physically by, e.g., stabilization of a discernible symptom, physiologically by, e.g., stabilization of a physical parameter, or both. In other embodiments the therapeutic treatment includes the reduction or stabilization of a disease, disorder or condition.

As used herein, the terms "subject" and "patient" are used interchangeably. The terms "subject" and "patient" refer to an animal (e.g., a bird such as a chicken, quail or turkey, or a mammal), specifically a "mammal" including a non-primate (e.g., a cow, pig, horse, sheep, rabbit, guinea pig, rat, cat, dog, and mouse) and a primate (e.g., a monkey, chimpanzee and a human), and more specifically a human. In one embodiment, the subject is a human.

The terms "severely inflamed" and "dysplastic" are interchangeable and in this specification, the terms "inflamed", "severely inflamed" and "dysplastic" are all interchangeable.

In this specification, the terms "malignant" and "neoplasic" are interchangeable. It will be understood that a neoplasia arises in the absence of inflammation, such as a spontaneous cancer. However, dysplasia occurs during inflammation etc. and leads to a malignancy. In addition, reference to carcinoma will be understood to arise from both a malignancy or neoplasia. Colon cancer may be referred to as colorectal cancer (CRC) or carcinoma. Colon cancer, breast cancer and oesophageal cancers are all adenocarcinomas.

In this manner, the invention as a whole is directed to the diagnosis and/or treatment of precancerous lesions, dysplasias, metaplasias and tumor diseases, including malignant neoplasias and metastasis, preferably solid tumors, particularly epithelial tumors. of the and GI tract.

In this specification, the cancer referred to is preferably an epithelial cell cancer, namely a carcinoma and preferably an adenocarcinoma which represents the largest group of carcinomas of the GI tract.

According to a general aspect of the invention, there is provided an in vitro method for the diagnosis and/or detection of GI tract carcinoma, preferably adenocarcinoma, comprising the steps of
a. monitoring and/or detecting the level of expression of caspase-4 and/or caspase-5 in a GI tract epithelial tissue and/or cells from a subject sample or biopsy;
b. comparing the level of expression of caspase-4 and/or caspase-5 in the sample or biopsy to the level of expression of caspase-4 and/or caspase-5 from a normal (negative) control and/or a positive control; and
c. identifying and/or diagnosing GI tract carcinoma when a detectable level or positive deviation of the expression levels of caspase-4 and/or caspase-5 compared to a normal (negative) control and/or a positive control is present within the GI tract epithelial tissue and/or cells sample or biopsy.

According to this general aspect, the present invention provides an in vitro method for the diagnosis and/or detection of GI tract carcinoma, comprising the steps of
a. monitoring and/or detecting the level of expression of caspase-4 and/or caspase-5 in a and GI tract epithelial tissue and/or cells from a subject sample or biopsy;
b. comparing the level of expression of caspase-4 and/or caspase-5 in the sample or biopsy to the level of expression of caspase-4 and/or caspase-5 from a normal (negative) control and/or a positive control; and
c. identifying and/or diagnosing and GI tract carcinoma when a detectable level or positive deviation of the expression levels of caspase-4 and/or caspase-5 compared to a normal (negative) control and/or a positive control is present within the and GI tract epithelial tissue and/or cells sample or biopsy.

According to one embodiment of this aspect of the invention, there is provided an in vitro method method for the identification of inflamed or malignant intestinal tissue and/or associated disease state, comprising the steps of
a. monitoring and/or detecting the level of expression of caspase-4 and/or caspase-5 in an intestinal tissue sample or a stool sample comprising intestinal cells, preferably intestinal epithelial and/or stromal intestinal cells, from a subject;
b. comparing the level of expression of caspase-4 and/or caspase-5 in the sample to the level of expression of caspase-4 and/or caspase-5 from a normal (negative) control and/or a positive control; and
c. identifying inflamed or malignant intestinal tissue and/or associated disease state when a detectable level or positive deviation (increased or upregulated expression) of the expression levels of caspase-4 and/or caspase-5 compared to a normal (negative) control and/or a positive control is present within the intestinal tissue sample or a stool sample.

In this aspect, the associated disease state may be a cancer or carcinoma.

This method enables the identification of inflamed intestinal tissue and/or associated disease state selected from inflammatory bowel disease (IBD), Crohn's disease (CD), ulcerative colitis (UC).when a detectable level or positive deviation of the expression levels of caspase-4 and/or caspase-5 compared to a normal (negative) control and/or a positive control is present within the intestinal stromal tissue sample or a stool sample.

This method also enables the identification of malignant intestinal tissue and/or associated disease state, including early stage adenoma and/or colorectal cancer (CRC), when a detectable level or positive deviation of the expression levels of caspase-4 and/or caspase-5 compared to a normal (negative) control and/or a positive control is present within the intestinal epithelial tissue sample or a stool sample.

According to this embodiment, there is provided an in vitro method for the identification and early diagnosis of IBD, including Crohn's disease (CD) and/or ulcerative colitis (UC), wherein detectable, increased or upregulated expression of caspase-4 and/or caspase-5 in non-inflamed stromal cells from the subject sample, compared to the level of expression of caspase-4 and/or caspase-5 in stromal cells from a normal control, is indicative of IBD, including CD and/or UC.

According to an embodiment of the invention, there is provided an in vitro method for the identification and early diagnosis of colorectal cancer (CRC) wherein detectable, increased or upregulated expression of caspase-4 and/or caspase-5 levels in epithelial cells from the subject sample, compared to the level of expression of caspase-4 and/or caspase-5 in epithelial cells from a normal control, is indicative of the presence of early stage CRC.

Also disclosed is a kit or array for preparing a caspase-4 and/or caspase-5 nucleic acid profile comprising probe sets designed to target caspase-4 and/or caspase-5, selected from one or more of probe sets SEQ ID Nos. 3 and 4, 5 and 6, 9 and 10, 11 and 12, 15 and 16, 17 and 18, 21 and 22, 23 and 24.

According to another aspect of the invention, there is provided caspase-4 and/or caspase-5 inhibitors for use in the treatment of GI tract carcinoma in a subject in need thereof, the use comprising administering a therapeutically effective amount of a caspase-4 and/or caspase-5 inhibitor to inhibit the expression of caspase-4 and/or caspase-5 in a subject. Also disclosed is a method for the treatment or prevention of intestinal inflammation in a subject comprising the step of inhibiting the expression of caspase-4 and/or caspase-5 in a subject.

According to a yet another aspect of the invention, there is provided caspase-4 and/or caspase-5 inhibitors for use in the treatment of GI tract carcinoma. Also disclosed is caspase-4 and/or caspase-5 inhibitors for use in the treatment and/or prevention of intestinal inflammation in a subject.

Also disclosed is caspase-4 and/or caspase-5 inhibitors or caspase-1 modulators/recombinant caspase-1 for use in the manufacture of a medicament for the treatment and/or prevention of carcinoma. A preferred disclosure is caspase-4 and/or caspase-5 inhibitors or caspase-1 modulators/ recombinant caspase-1 for use in the manufacture of a medicament for the treatment and/or prevention of intestinal inflammation in a subject.

According to another aspect of the invention, there is provided an in vitro method for the treatment GI tract carcinoma, comprising the steps of
a. providing epithelial tissue and/or cells from a subject sample or biopsy;
b. monitoring and/or detecting the level of expression of caspase-4 and/or caspase-5 in epithelial tissue and/or cells from a subject sample or biopsy;
c. comparing the level of expression of caspase-4 and/or caspase-5 in the sample or biopsy to the level of expression of caspase-4 and/or caspase-5 from a normal (negative) control and/or a positive control; and
d. identifying and/or diagnosing malignancy when a detectable level or positive deviation of the expression levels of caspase-4 and/or caspase-5 compared to a normal (negative) control and/or a positive control is present within the epithelial tissue and/or cells sample or biopsy;
Also disclosed is treating the subject when diagnosed with malignancy, with conventional cancer treatment, including radiotherapy or chemotherapy.

According to another aspect of the invention, there is provided a caspase-1 modulator to increase caspase-1 expression levels or recombinant caspase-1 for use in the treatment of and GI tract carcinoma.

Also disclosed is a method for the treatment and/or prevention of carcinoma and/or inflammation in a subject in need thereof, comprising administering a therapeutically effective amount of a caspase-1 modulator or recombinant caspase-1 to increase the expression of caspase-1 in a subject.

According to another aspect of the invention, there is provided a caspase-1 modulator to increase caspase-1 expression levels or recombinant caspase-1 for use in the treatment of GI tract carcinoma, comprising the steps of
a. providing epithelial tissue and/or cells from a subject sample or biopsy;
b. monitoring and/or detecting the level of expression of caspase-1 in epithelial tissue and/or cells from a subject sample or biopsy;
c. comparing the level of expression of caspase-1 in the sample or biopsy to the level of expression of caspase-1 from a normal (negative) control and/or a positive control; and
d. identifying and/or diagnosing malignancy when a detectable level or negative deviation of the expression levels of caspase-1 compared to a normal (negative) control and/or a positive control is present within the epithelial tissue and/or cells sample or biopsy;

Also disclosed is treating the subject when diagnosed with malignancy, with conventional cancer treatment, including radiotherapy or chemotherapy.

### DETAILED DESCRIPTION OF THE INVENTION

### CARCINOMAS

The invention is based on our findings that normal epithelial cells do not express caspase-4 and/or - 5, however, we have found that malignant (neoplasic) epithelial cells express caspase-4 and/or-5. This differential expression of caspase-4 and/or -5 can advantageously be used for the diagnosis and detection of carcinomas, in particular early stage carcinomas. We have also found that caspase-1 expression is downregulated as tumor grade/malignancy progresses.

According to a general aspect of the invention, there is provided an in vitro method for the diagnosis and/or detection of GI tract carcinoma, preferably adenocarcinoma, comprising the steps of
a. monitoring and/or detecting the level of expression of caspase-4 and/or caspase-5 in a and GI tract epithelial tissue and/or cells from a subject sample or biopsy;
b. comparing the level of expression of caspase-4 and/or caspase-5 in the sample or biopsy to the level of expression of caspase-4 and/or caspase-5 from a normal (negative) control and/or a positive control; and
c. identifying and/or diagnosing GI tract carcinoma when a detectable level or positive deviation of the expression levels of caspase-4 and/or caspase-5 compared to a normal (negative) control and/or a positive control is present within the epithelial tissue and/or cells sample or biopsy.

According to this general aspect, the present invention provides an in vitro method for the diagnosis and/or detection of GI tract carcinoma, comprising the steps of
a. monitoring and/or detecting the level of expression of caspase-4 and/or caspase-5 in a GI tract epithelial tissue and/or cells from a subject sample or biopsy;
b. comparing the level of expression of caspase-4 and/or caspase-5 in the sample or biopsy to the level of expression of caspase-4 and/or caspase-5 from a normal (negative) control and/or a positive control; and
c. identifying and/or diagnosing GI tract carcinoma when a detectable level or positive deviation of the expression levels of caspase-4 and/or caspase-5 compared to a normal (negative) control and/or a positive control is present within the GI tract epithelial tissue and/or cells sample or biopsy.

Advantageously, the invention enables the early diagnosis and/or detection of such carcinomas. This is advantageous when histology results are difficult to diagnose, for example under circumstances where significant levels of inflammation exist within the tissue. Pathologists often have difficulty differentiating between inflamed tissue and early stage cancer tissue. Based on these findings, which show elevated expression levels of caspases-4 and -5 in malignant epithelial cells, approaches complementary to H&E staining, which will examine epithelial caspase-4 and/or -5 expression, may be employed to assist in carcinoma diagnosis and/or detection.

According to one embodiment, the method of the invention provides for early diagnosis and/or detection of carcinoma where increased caspase-5 expression levels provide for early diagnosis and/or detection of carcinoma.

According to another embodiment, the method provides for the grading and/or staging of carcinoma where caspase-1 expression levels decrease with increasing tumor grade and/or caspase-4 expression levels increase with increasing tumor grade

Ideally, the sample is a biopsy or resection tissue and may comprise a cross-section of tissue from epithelial, stromal and possibly other cell layers. In this manner, the sample comprises at least epithelial tissue and/or cells from a subject.

Optionally, the level of expression of caspase-1, caspase-4 and/or caspase 5 in stromal tissue and/or cells is also measured and increased expression levels are indicative of tissue inflammation.

We have also observed that caspase-1 levels can be indicative of tumour cell progression and/or staging. In this manner, one embodiment of the invention comprises the steps of
a. monitoring and/or detecting the level of expression of caspase-1 in epithelial tissue and/or cells from a subject sample or biopsy;
b. comparing the level of expression of caspase-1 in the sample or biopsy to the level of expression of caspase-1 from a normal (negative) control and/or a positive control; and
c. identifying and/or diagnosing malignancy when a detectable level or negative deviation of the expression levels of caspase-1 compared to a normal (negative) control and/or a positive control is present within the epithelial tissue and/or cells sample or biopsy.

We have shown that caspase-1 expression levels decrease with increasing tumor grade and/or caspase-4 expression levels increase with increasing tumor grade. These results can allow more accurate staging of the progression of the tumour grade, for example from T1 to T3 and optionally T4.

In this manner caspase-1 staining/expression level analysis can be used in combination with caspase-4 and/or -5 staining/expression level analysis to assess the stage/progression of the malignancy or tumour.

The carcinoma may be selected from an adenocarcinoma, squamous cell carcinoma, basal cell carcinoma or transitional cell carcinoma. Optionally, according to any of the embodiments of the invention the carcinoma excludes colorectal cancer. Still optionally, according to any of the embodiments of the invention the carcinoma excludes oesophageal adenocarcinoma (OAC).

The carcinoma is ideally present in the GI tract, and is preferably oesophageal adenocarcinoma (OAC) or colorectal cancer.

The GI tract carcinoma is ideally selected from cancers of the oesophagus, stomach, biliary system, pancreas, small intestine, large intestine (colon), rectum and anus. Optionally the GI tract carcinoma excludes colorectal cancer.

We have advantageously shown that caspase-1, caspase-4 and caspase-5 expression levels provide a valuable diagnostic for many different types of carcinomas, including colorectal cancer (CRC) and oesophageal adenocarcinoma (OAC).

According to another aspect of the invention, there is provided caspase-4 and/or caspase-5 inhibitors for use in the treatment of GI tract carcinoma, comprising the steps of
a. providing epithelial tissue and/or cells from a subject sample or biopsy;
b. monitoring and/or detecting the level of expression of caspase-4 and/or caspase-5 in epithelial tissue and/or cells from a subject sample or biopsy;
c. comparing the level of expression of caspase-4 and/or caspase-5 in the sample or biopsy to the level of expression of caspase-4 and/or caspase-5 from a normal (negative) control and/or a positive control; and
d. identifying and/or diagnosing malignancy when a detectable level or positive deviation of the expression levels of caspase-4 and/or caspase-5 compared to a normal (negative) control and/or a positive control is present within the epithelial tissue and/or cells sample or biopsy

Also disclosed is treating the subject when diagnosed with malignancy, with conventional cancer treatment, including radiotherapy or chemotherapy.

These finding have led to potential treatments for carcinoma. According to one aspect, caspase-4 and/or caspase-5 inhibitors are provided for use in the treatment and/or prevention of carcinoma. The carcinoma may be an adenocarcinoma.

Ideally, the caspase-4 and/or caspase-5 inhibitors target and inhibit or reduce caspase-4 and/or caspase-5 expression in epithelial tissues and/or cells in a subject.

Another aspect is caspase-4 and/or caspase-5 inhibitors for use in the treatment of GI tract carcinoma in a subject in need thereof, the use comprising administering a therapeutically effective amount of a caspase-4 and/or caspase-5 inhibitors to inhibit the expression of caspase-4 and/or caspase-5 in a subject.

It will be understood that the caspase-4 and/or caspase-5 inhibitor may an siRNA targeting caspase-4 and/or caspase-5.

As caspase-1 levels decrease with increasing tumour grade, we postulate that the use of a modulator to increase caspase-1 expression and/or activity in epithelial cells will have an anti-tumorigenic impact. Accordingly, another aspect is a caspase-1 modulator or recombinant caspase-1 to increase caspase-1 expression levels for use in the treatment and/or prevention of GI tract carcinoma.

Yet another aspect is a caspase-1 modulator to increase caspase-1 expression levels or reconbinant caspase-1 for use in the treatment of GI tract carcinoma in a subject in need thereof, the use comprising administering a therapeutically effective amount of a caspase-1 modulator or recombinant caspase-1 to increase the expression of caspase-1 in a subject.

Alternatively, instead of a caspase-1 modulator, recombinant caspase-1 or the products (downstream products) of caspase-1 activity, such as IL-18 or IL-1beta, may be delivered using conventional means (e.g. via electroporation, microinjection or macromolecular systems) to epithelial cells, such as CRC epithelial cells.

These methods represent novel therapies to delay the progression of carcinoma.

### COLORECTAL CANCER (CRC)

Our findings have shown that caspase-1, caspase-4 and/or caspase-5 expression levels provide a valuable diagnostic for colorectal cancer patients. In this manner, caspase-1, caspase-4 and/or caspase-5 expression levels can be used in assessing colorectal cancer progression in an individual. Unlike caspase-1, caspase-4 and caspase-5 are not expressed in normal epithelial cells. Our findings show that any detectable level of either caspase-4 or -5 in epithelial cells is indicative of cancer.

We have found that caspase-1 and caspase-4 expression levels within intestinal epithelial cells (IECs) of CRC patients is indicative of tumour grade, for example from T1 (lower grade tumours) to T2/T3 (advanced grade tumours) or T4. Caspase-1 levels have been found to decrease with increasing tumour grade (e.g. from T1 to T3 or T4). Caspase-4 levels have been found to increase with increasing tumour grade (e.g. from T1 to T3 or T4).

We have also found that caspase-5 expression levels in IECs are elevated across all tumour grades. Our findings shown that caspase-5 expression levels are maximally elevated even at T1, the earliest tumour stage, when tumour growth has not spread beyond the inner layer of the colon. For these reasons, caspase-5 can advantageously provide early stage tumour detection.

According to this embodiment, there is provided an in vitro method method for the diagnosis and/or detection of colorectal cancer (CRC) according to claim 1, comprising the steps of
a. monitoring and/or detecting the level of expression of caspase-4 and/or caspase-5 in a and GI tract epithelial tissue and/or cells from a subject sample or biopsy;
b. comparing the level of expression of caspase-4 and/or caspase-5 in the sample or biopsy to the level of expression of caspase-4 and/or caspase-5 from a normal (negative) control and/or a positive control; and
c. identifying and/or diagnosing CRC when a detectable level or positive deviation of the expression levels of caspase-4 and/or caspase-5 compared to a normal (negative) control and/or a positive control is present within the and GI tract epithelial tissue and/or cells sample or biopsy.

Advantageously, wherein increased caspase-5 expression levels provide for early diagnosis and/or detection of carcinoma.

Caspase-1 levels may also be monitored in a similar manner and CRC may be identified and/or diagnosed when a negative deviation of the expression levels of caspase-1 compared to a normal (negative) control and/or a positive control is present within the epithelial tissue and/or cells sample or biopsy.

Advantageously, the tumour stage or progression may be monitored using this method. We have found that caspase-1 expression levels decrease with increasing tumor grade and/or caspase-4 expression levels increase with increasing tumor grade.

These findings have also led to potential therapies for carcinoma.

According to one embodiment, caspase-4 and/or caspase-5 inhibitors may be used in the treatment of GI tract carcinoma.

The carcinoma may be an adenocarcinoma. Optionally, the carcinoma is a and GI tract cancer optionally selected from cancers of the oesophagus, stomach, biliary system, pancreas, small intestine, large intestine, rectum and anus, colorectal cancer (CRC) or early stage adenoma in a subject. Preferably, the carcinoma is colorectal cancer (CRC), breast carcinoma or oesophageal carcinoma.

In this manner the caspase-4 and/or caspase-5 inhibitors target and inhibit or reduce caspase-4 and/or caspase-5 expression in epithelial tissues and/or cells in a subject. For example, the caspase-4 and/or caspase-5 inhibitor may be an siRNA targeting caspase-4 and/or caspase-5.

Advantageously, the caspase-4 and/or caspase-5 inhibitors may be used in the treatment of early stage carcinoma.

According to another embodiment, there is provided caspase-4 and/or caspase-5 inhibitors for use in the treatment of GI tract carcinoma in a subject in need thereof, the use comprising administering a therapeutically effective amount of a caspase-4 and/or caspase-5 inhibitors to inhibit the expression of caspase-4 and/or caspase-5 in a subject.

Again, the caspase-4 and/or caspase-5 inhibitor may be an siRNA targeting caspase-4 and/or caspase-5.

The carcinoma may be an adenocarcinoma. Optionally, the carcinoma is a GI tract cancer optionally selected from cancers of the oesophagus, stomach, biliary system, pancreas, small intestine, large intestine, rectum and anus, colorectal cancer (CRC) or early stage adenoma in a subject. Preferably, the carcinoma is colorectal cancer (CRC), breast carcinoma or oesophageal carcinoma.

The subject is ideally a mammal, preferably a human.

According to another embodiment, there is provided a caspase-1 modulator to increase caspase-1 expression levels or recombinant caspase-1 for use in the treatment and/or prevention of carcinoma.

The carcinoma may be an adenocarcinoma. Optionally, the carcinoma is a GI tract cancer optionally selected from cancers of the oesophagus, stomach, biliary system, pancreas, small intestine, large intestine, rectum and anus, colorectal cancer (CRC) or early stage adenoma in a subject. Preferably, the carcinoma is colorectal cancer (CRC), breast carcinoma or oesophageal carcinoma.

In this manner the caspase-4 and/or caspase-5 inhibitors target and inhibit or reduce caspase-4 and/or caspase-5 expression in epithelial tissues and/or cells in a subject. For example, the caspase-4 and/or caspase-5 inhibitor may be an siRNA targeting caspase-4 and/or caspase-5.

According to another embodiment, there is provided a caspase-1 modulator to increase caspase-1 expression levels or recombinant caspase-1 for use the treatment of GI tract carcinoma in a subject in need thereof, the use comprising administering a therapeutically effective amount of a caspase-1 modulator or recombinant caspase-1 to increase the expression of caspase-1 in a subject.

The carcinoma may be an adenocarcinoma. Optionally, the carcinoma is a GI tract cancer optionally selected from cancers of the oesophagus, stomach, biliary system, pancreas, small intestine, large intestine, rectum and anus, colorectal cancer (CRC) or early stage adenoma in a subject. Preferably, the carcinoma is colorectal cancer (CRC), breast carcinoma or oesophageal carcinoma.

The subject is ideally a mammal, preferably a human.

### INFLAMMATORY BOWEL DISEASE (IBD)

According to a general aspect of the invention there is provided an in vitro method for the identification of inflamed or malignant intestinal tissue and/or associated disease state, comprising the steps of
a. monitoring and/or detecting the level of expression of caspase-4 and/or caspase-5 in an intestinal tissue sample or a stool sample comprising intestinal cells, preferably intestinal and/or stromal intestinal cells, from a subject;
b. comparing the level of expression of caspase-4 and/or caspase-5 in the sample to the level of expression of caspase-4 and/or caspase-5 from a normal (negative) control and/or a positive control; and
c. identifying inflamed or malignant intestinal tissue and/or associated disease state when a detectable level or positive deviation (increased or upregulated expression) of the expression levels of caspase-4 and/or caspase-5 compared to a normal (negative) control and/or a positive control is present within the intestinal tissue sample or a stool sample.

In this manner, inflamed intestinal tissue and/or cells is generally associated with IBD and related disorders. Malignant intestinal tissue and/or cells is generally associated with carcinoma such as colorectal cancer or adenoma.

Our studies on the expression of caspases-1, -4 and -5 in healthy, IBD and CRC (both spontaneous and IBD-associated CRC) patients have yielded some unexpected results. We have surprisingly found that elevated expression of caspases-4 and-5 in intestinal tissue samples can predict IBD, and furthermore, that the differential expression of caspases-4 and -5 in epithelial and stromal cell layers can be advantageously used in the identification of colorectal cancer, malignant intestinal tissue and/or associated disease progression from inflammatory bowel disease to cancer. Using IHC techniques we have found that the expression of inflammatory caspases in intestinal epithelial and stromal cells can be utilised in new diagnostic methods.

Specifically, we have surprisingly found out that expression of caspase-4 and caspase-5 within infiltrating inflammatory cells of stromal tissue indicates IBD, however, expression in epithelial cells indicates malignancy, and the presence of CRC. These unexpected findings allow the improved distinction of several colon diseases associated with inflammation by using specific cells for expression analysis. This advantageously leads to a method for allowing the differentiation between inflamed IBD tissue and early stage CRC tissue.

We have unexpectedly found that the expression of caspase-4 and -5 within inflammatory cells of stromal tissue (stromal expression) strongly correlate with the levels of inflammation and disease activity in IBD patients, whereas caspase-1 expression levels did not exhibit such clear correlations. This allows us to identify inflamed intestinal tissue and/or associated disease state including inflammatory bowel disease (IBD), Crohn's disease (CD), ulcerative colitis (UC).

We have also found that caspase-4 and -5 expression only occurs in malignant intestinal epithelial cells (arising from either neoplasia or dysplasia). Epithelial cells from healthy, and even severely inflamed patients, are negative for caspase-4 and -5 expression. This is unexpected, as caspase-1 is constitutively expressed in epithelial cells from healthy and inflamed patients, and also in contrast to caspases-4 and -5, caspase-1 expression levels decrease in malignant epithelial cells. The important implication from these findings is for CRC diagnosis, as malignant epithelial cells can be identified immediately and early if found to be positive for caspase-4/-5 expression. This allows us to diagnosis malignant intestinal tissue and/or associated disease state including early stage adenoma and colorectal cancer (both spontaneous and colitis-associated CRC).

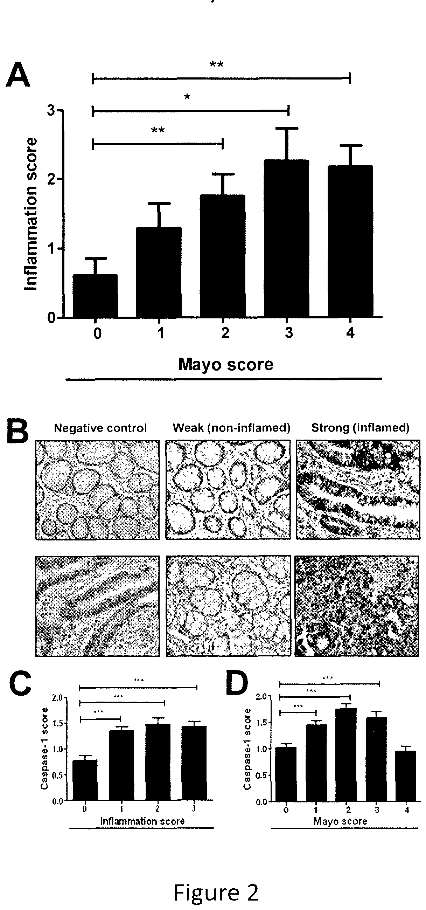

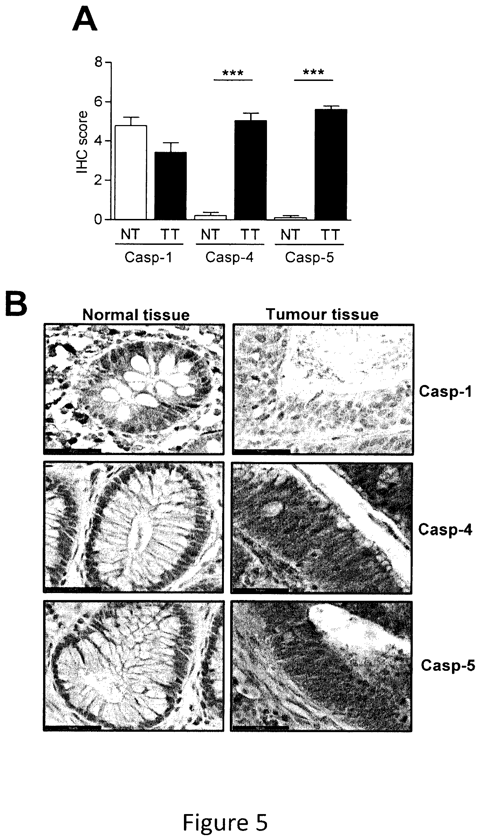

Our invention utilises these findings that caspases-4 and -5 are exclusively expressed in cells within stromal tissue (stromal cells) until malignancy, when they also become robustly expressed in epithelial cells. In contrast, caspase-1 is expressed in both stromal and epithelial cells, even in healthy patients. Although caspase-1 expression also increases in both epithelial and stromal cell types during inflammation (fig.2), it differs to caspase-4 and -5 as it is not upregulated further in malignant epithelial cells (fig.5). We have found that caspase-1 expression levels decrease with increasing tumor grade, thus, caspase-1 expression levels assist in the grading and/or staging of CRC.

Thus, despite the findings that mutations in inflammatory caspases are not causally implicated in oncogenesis, we have surprisingly found that
- Quantitative PCR identified statistically significant increases in expression of inflammatory caspase-1, -4 and -5 within inflamed tissue from IBD patients (Kruskal Wallis statistical test with Dunn's post hoc comparison to control).Quantitative PCR also identified statistically significant increased levels of caspase-4 in non-inflamed tissue of IBD patients compared to healthy control, suggesting that caspase-4 may be an early marker of IBD (Kruskal Wallis statistical test with Dunn's post hoc comparison to control).
- Using IHC staining techniques, we have also elucidated a significant correlation between stromal caspase-4 and -5 expression levels, inflammation and disease activity in UC patients (Mann Whitney statistical tests). Epithelial expression of caspase-4 or -5 could not be detected in normal or inflamed tissue from healthy or IBD patients.
- We have also found significantly increased stromal expression of caspases-4 and -5 in tumor tissue, compared with adjacent normal tissue, of CRC patients (Mann Whitney statistical tests)
- Surprisingly, we identified robust expression of caspases-4 and -5 within intestinal epithelial cells, exclusively within neoplastic tissue, of colorectal tumors
- An examination of adjacent-normal, inflamed and tumor tissue from patients with UC-associated CRC confirmed that stromal expression of caspases-4 and -5 is increased in inflamed and malignant tissue, while epithelial expression is restricted to malignant tissue.
- Screening a panel of epithelial cell lines confirmed that caspases-4 and -5 are expressed in all CRC epithelial cell lines examined to date.
- We postulate that siRNA based 'silencing' of caspase-4 and caspase-5 in CRC epithelial cells will impact on cell proliferation, cell death, cell migration OR other inflammatory/carcinogenic cellular functions.
- Based on their increased expression in stromal cells of inflamed UC/CRC patient tissue, caspase-4 and -5 have been identified as novel targets for the clinical management of intestinal inflammation.

It will be understood that the sample is ideally an intestinal tissue sample or a stool sample comprising intestinal cells from a subject. The sample itself may be obtained from the subject as a tissue sample or biopsy. The subject is a mammal, preferably a human.

According to one embodiment the sample is an intestinal tissue sample. Ideally, the intestinal tissue sample is a stromal tissue sample or biopsy and/or an epithelial tissue sample or biopsy.

According to another embodiment the sample is a stool sample comprising intestinal cells from a subject. The entire epithelial cell layer of the intestine is replaced every 4 to 7 days. Thus, due to epithelial cell shedding, a stool sample will ideally comprise intestinal epithelial cells and will, for example, allow qPCR-based detection of caspase-4 or -5 from malignant epithelial cells.

It will be understood that the sample may be from a subject which is a healthy individual, an individual with a family history of Inflammatory Bowel Disease (IBD) or colorectal cancer (CRC), or an individual suffering from IBD, CD or ulcerative colitis (UC). Thus, the sample may comprise non-inflammed, inflamed, malignant, dysplastic and/or neoplastic, intestinal cells derived from the subject.

Such malignant intestinal tissue may arise sporadically in otherwise healthy patients. Alternatively, the malignant intestinal tissue may be as a result of intestinal inflammation in patients diagnosed with Inflammatory bowel disease, who are also at increased risk of developing colorectal cancer.

Advantageously, the present invention enables the differentiation between healthy, inflamed or malignant, including dysplastic and/or neoplastic, intestinal tissue. At present, pathologists analyse biopsy/resection tissue using standard H&E staining techniques to observe the tissue histology, but it is often very difficult to distinguish between inflamed and dysplastic/neoplastic tissue by this method. This type of analysis can unfortunately result in delayed or misdiagnosis of disease states. Thus, a significant advantage of the invention is that the identification and diagnosis of CRC tissue will be easier and more sensitive than current methods. The use of stool samples for screening also gives the advantage that CRC screening can take place at an earlier stage than previously possible. In fact, the present invention enables the early diagnosis of both IBD and CRC.

We have found that caspases-4 and -5 are novel discriminators between inflamed and neoplastic tissue, and suggest that there may be significant scope for the detection of epithelial caspase-4 and - 5 as determinants of malignancy within colorectal mucosa. The presence of caspase-4 and -5 in epithelial cells could be developed as a complimentary marker to histological assessment of dysplasia within colon tissue when predicting CRC risk in patients.

Thus, the method of the invention enables the early identification of inflamed or malignant intestinal tissue and/or associated disease state. The method of the invention has recognized that the increased or upregulated expression of caspase-4 and/or caspase-5 from a normal control is indicative of a malignant intestinal tissue progression or associated disease progression phenotype.

In this specification, it will be understood that the terms "positive deviation" embraces and is interchangeable with the terms "detectable", "increased or upregulated expression". Thus, a positive deviation in terms of detectable level, increased or upregulated expression of caspase-4 and/or caspase-5, compared to background expression of caspase-4 and -5 in, for example, normal (negative) controls and/or a positive control, have been found to be indicative of inflamed or malignant (i.e. dysplastic/neoplastic) intestinal tissue.

According to one embodiment of the invention, the method enables the early identification of inflammatory bowel disease (IBD) wherein increased or upregulated expression of caspase-4 and/or caspase-5 is indicative of early stage Inflammatory Bowel Disease (IBD). Specifically, the method enables the identification and early diagnosis of IBD, including Crohn's disease (CD) and/or ulcerative colitis (UC), wherein increased or upregulated expression of caspase-4 and/or caspase-5 in non-inflamed intestinal stromal cells from the subject sample, compared to the level of expression of caspase-4 and/or caspase-5 in intestinal stromal cells from a normal control, is indicative of IBD, including CD and/or UC. In this manner, we have found that the extent of inflammation and increase in disease activity, compared to background expression of caspase-4 and -5, correlates with increased caspase-4 and -5 expression in intestinal stromal tissue and/or cells.

According to another embodiment of the invention, we have surprisingly found that intestinal epithelial expression of caspases-4 and -5 is restricted to neoplastic tissue. Thus, the method enables the identification and early diagnosis of CRC wherein a positive deviation in terms of a detectable, increased or upregulated expression of caspase-4 and/or caspase-5 levels in intestinal epithelial cells from the subject sample, compared to the level of expression of caspase-4 and/or caspase-5 in intestinal epithelial cells from a normal control, is indicative of the presence of early stage CRC. In this manner, we have found that a detectable level of caspase-4 and -5 within epithelial tissue and/or cells is indicative of CRC whereas normal surrounding tissue has no detectable levels of caspase-4 and -5, thereby allowing a positive diagnosis of CRC.

In all aspects of the invention, Various techniques may be used to monitor and measure the expression of caspase-1, caspase-4 and caspase-5. These methods include qPCR, immunofluorescence (IF), ELISA and/or immunohistochemistry (IHC). It will also be understood that the expression level of caspase-1 caspase-4 and/or caspase-5 is determined by evaluating the abundance of RNA transcripts transcribed from the gene, evaluating the abundance of polypeptides translated from said RNA transcripts, or a combination thereof. For example, the expression level of caspase-4 and/or caspase-5 is determined by quantifying a functional RNA transcript.

According to one embodiment of the invention a positive deviation of the expression level of more than 1-fold, optionally more than 1.5-fold, further optionally more than 2-fold, further optionally more than 3-fold, further optionally more than 4-fold, of the level of caspase-4 and/or caspase-5 expression from a normal control is generally indicative of malignancy, malignant intestinal tissue progression or associated disease progression phenotype.

Specifically, caspase-4 levels increase more than 1-fold, optionally more than 1.5-fold, further optionally more than 2-fold, further optionally more than 3-fold, further optionally more than 4-fold as the tumour stage increases (T1 to T3 and optionally T4). For these reasons, caspase-4 levels can be used to determine tumour progression and/or staging.

Our findings indicate that caspase-5 is maximally expressed at early tumour stage (T1), thereby indicating malignancy. Thus, increased levels do not really indicate progression of the malignancy. For these reasons, we believe that caspase-5 can be used of the early diagnosis of malignancy.

It will be understood that, for example, within intestinal epithelial cells it is the detection of any caspase-4 or -5 which indicates malignancy (dysplasia/neoplasia). In epithelial cells any value above background is indicative of malignancy. For example when IHC is used, a score greater than 1 is indicative of malignancy.

Immunohistochemistry (IHC) or immunoflourescence (IF) may be used to identify epithelial cells and/or stromal cells in the sample and to detect the level of expression the level of expression of caspase-1, caspase-4 and/or caspase-5 within the epithelial tissue and/or cells and/or stromal tissue and/or cells.

It will be understood that the normal control is associated with a disease-free phenotype. Alternatively, it will be understood that the normal control is associated with a defined stage of malignant intestinal tissue progression or associated disease progression.

It will be understood that the positive control is associated with a disease phenotype, for example a subject with colorectal cancer (CRC).

It will be understood that the intestinal tissue sample is a stromal tissue sample or biopsy and/or an epithelial tissue sample or biopsy.

It will be understood that the disease state may be inflammatory bowel disease (IBD), Crohn's disease (CD), ulcerative colitis (UC) and/or colorectal cancer (CRC), including spontaneous CRC and colitis-associated CRC.

According to another embodiment of the invention, the expression level of caspase-4 and/or caspase-5 is determined using polynucleotides having a nucleic acid sequence capable of hybidising to some, optionally all, of the nucleic acid sequences selected from probe sets SEQ ID Nos. 3 and 4, 5 and 6, 9 and 10, 11 and 12, 15 and 16, 17 and 18, 21 and 22, 23 and 24.

Also disclosed is a kit or array for preparing a caspase-4 and/or caspase-5 nucleic acid profile associated comprising probe sets designed to target caspase-4 and/or caspase-5, selected from one or more of probe sets SEQ ID Nos. 3 and 4, 5 and 6, 9 and 10, 11 and 12, 15 and 16, 17 and 18, 21 and 22, 23 and 24.

Full details of the primers used to target caspase-1, -4 and -5 are given in the table below.

| **SEQUENCE** | **SEQUENCE NAME (F-forward R - reverse)** | **SEQ ID NO** |
|---|---|---|
| **Primer set 1 (****Figure 1****)** | | |
| TGCCTGTTCCTGTGATGTGGAGGA | CASP1F | SEQ ID NO 1 |
| CAGTGGTGGGCATCTGCGCT | CASP1R | SEQ ID NO 2 |
| ACCGTGGGGAACTGTGGGTCA | CASP4F | SEQ ID NO 3 |
| CCAGGACACGTTGTGTGGCGT | CASP4R | SEQ ID NO 4 |
| CGCAGACGCCTGGCTCTCAT | CASP5F | SEQ ID NO 5 |
| AGCCCAGGCCTTGAAGCAGC | CASP5R | SEQ ID NO 6 |

| **Primer set 2** | | |
|---|---|---|
| ATCCGTTCCATGGGTGAAGG | CASP16F | SEQ ID NO 7 |
| GCCCCTTTCGGAATAACGGA | CASP16R | SEQ ID NO 8 |
| TCTGAGGCTCTTTCCAACGC | CASP4αF | SEQ ID NO 9 |
| CCCAGGGATTCCAACACCTT | CASP4αR | SEQ ID NO 10 |
| AGATGTTGGAATACCTGGGCA | CASP5eF | SEQ ID NO 11 |
| TGATGAGCCACGCGATTCTT | CASP5eR | SEQ ID NO 12 |

| **Primer set 3** | | |
|---|---|---|
| GAAAAGCCATGGCCGACAAG | CASP1γF | SEQ ID NO 13 |
| AGGAGCTTCACCCATGGAAC | CASP1γR | SEQ ID NO 14 |
| TTCACCAACTGTCAGAAGGCA; | CASP4γF | SEQ ID NO 15 |
| GTCTGCCATGACCCGAACTT | CASP4γR | SEQ ID NO 16 |
| TTAGCTATGGCTGCTCTTCTGC | CASP5cF | SEQ ID NO 17 |
| GTCTGCGGTCCTCTCTCTTTTT | CASP5cR | SEQ ID NO 18 |

| **Primer set 4** | | |
|---|---|---|
| GTTCCATGGGTGAAGATAATGTTT | CASP1εF | SEQ ID NO 19 |
| GCATCTGCGCTCTACCATCT | CASP1εR | SEQ ID NO 20 |
| CTTCACCAACTGTCAGAAGGC | CASP4γF | SEQ ID NO 21 |
| TCTGCCATGACCCGAACTTT | CASP4γR | SEQ ID NO 22 |
| TGTTGGAATACCTGGGCAAAGA | CASP5eF | SEQ ID NO 23 |
| TTGATGAGCCACGCGATTCT | CASP5eR | SEQ ID NO 24 |

Also disclosed is a method for the treatment and/or prevention of intestinal inflammation in a subject comprising the step of inhibiting the expression of caspase-4 and/or caspase-5 in a subject. This method comprises the use of caspase-4 and/or caspase-5 inhibitors or siRNA directed to caspase-4 and/or caspase-5.

Also disclosed is caspase-4 and/or caspase-5 inhibitors for use in the treatment and/or prevention of intestinal inflammation in a subject, in particular inflammatory bowel disease (IBD), ulcerative colitis (UC) and/or colorectal cancer (CRC).

Also disclosed is the use of caspase-4 and/or caspase-5 inhibitors in the manufacture of a medicament for the treatment and/or prevention of intestinal inflammation in a subject.

According to an aspect of the invention, there is provided caspase-4 and/or caspase-5 inhibitors for use in the treatment and/or prevention of carcinoma in a subject in need thereof, the use comprising administering a therapeutically effective amount of a caspase-4 and/or caspase-5 inhibitors to inhibit the expression of caspase-4 and/or caspase-5 in a subject.

According to one embodiment, the inhibitors target and inhibit or reduce caspase-4 and/or caspase-5 expression in non-inflamed stromal cells in a subject.

According to another embodiment, the inhibitors target and inhibit or reduce caspase-4 and/or caspase-5 expression in epithelial cells in a subject.

According to all embodiments of the invention, the caspase-4 and/or caspase-5 inhibitors may be selected from naturally occurring caspase inhibitors or chemically synthesised peptide inhibitors.

Naturally occuring caspase inhibitors include virally derived caspase inhibitors such as CrmA from cowpox virus; and bacterially derived caspase inhibitors, such as OspC3 from Shigella. Chemically synthesised peptide inhibitors include short tri/tetra-peptides that mimic the amino acid sequences within substrates targeted by caspases. These peptide inhibitors act as competitive inhibitors and essentially 'mop up' all the active caspase so that they are no longer available to carry out their normal cellular function.

Optionally, the chemically synthesized peptide inhibitors are selected from peptides including z-VAD-fmk, YVAD-fmk, LSTD.fmk and/or WEHD-fmk.

Alternatively, the naturally occurring caspase inhibitor is a cowpox virus protein, including CrmA (Cytokine response modifier A) and OspC3.

Other conventional caspase inhibitors may be used in accordance with the invention.

The invention will now be described with respect to the following non-limiting figures and examples.

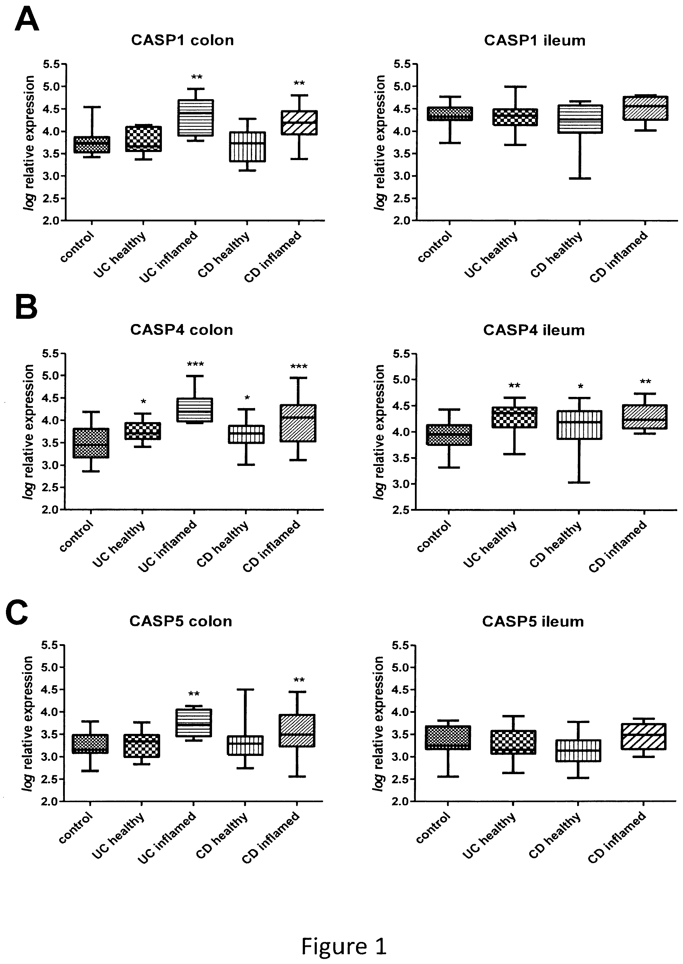

**Figure 1****: Increased inflammatory caspase gene expression in IBD patient biopsies from a Belgian cohort.**
   Relative expression of CASP1 (A), CASP4 (*B*) and CASP5 (*C*) in biopsy specimens from non-IBD healthy control (n=21), active UC patients in endoscopically healthy (n=10) and inflamed (n=11) areas, and active CD patients in healthy (n=38) and inflamed (n=21) colon and ileum as quantified by qPCR. Data are normalized to the median of 3 stably expressed reference genes and represent median and range; **p* < 0.05; ***p* < 0.01; ****p* < 0.001(Kruskal Wallis test with Dunn's post hoc comparison to control).
**Figure 2****: Inflammatory caspase-1 expression strongly correlates with clinical disease and inflammation scores in Ulcerative Colitis patient biopsies from an Irish cohort.**
   (A) Inflammation score correlates with endoscopic Mayo score (Disease activity score). Mayo score was graded on a scale of 0-4 (with (0) indicating no activity; (1) minimal activity; (2) mild activity; (3) moderate and (4) severe activity). Inflammation score was graded on a scale of 0-3 (with (0) indicating no activity; (1) mild activity; (2) moderate activity and (3) severe activity. (*B*) Representative images of caspase-1 expression pattern in epithelial cells (top panel) and stromal cells (bottom panel) from non-inflamed and inflamed UC biopsy sections. Negative staining was observed for isotype rabbit/mouse IgG control (negative control). Original magnification ×20. (*C*) Caspase-1 expression levels correlate with histological inflammatory scores for UC colonic biopsies. (*D*) Caspase-1 expression levels versus endoscopic Mayo score. IHC was performed on colon tissue from 11 non-inflamed UC patients and 25 patients with varying degrees of inflammation. Data represents mean ± SEM; **p* < 0.05; ***p* < 0.01, ****p* < 0.001 (Mann Whitney U test).

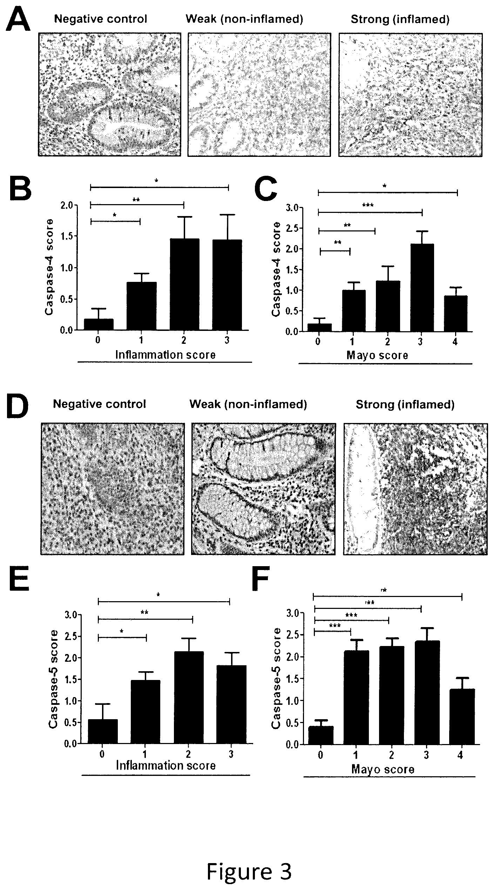

**Figure 3****: Inflammatory caspase-4 and -5 expression strongly correlates with clinical disease and inflammation scores in Ulcerative Colitis patient biopsies from an Irish cohort.**
   Representative images of (A) caspase-4 and (D) caspase-5 expression patterns in stromal cells of UC biopsy sections. Original magnification ×20. (B) caspase-4 and (E) caspase-5 expression levels correlate with histological inflammatory scores for UC colonic biopsies. Inflammation score was graded on a scale of 0-3 (with (0) indicating no activity; (1) mild activity; (2) moderate activity and (3) severe activity. (C) caspase-4 and (F) caspase-5 expression levels versus endoscopic Mayo score. Mayo score was graded on a scale of 0-4 (with (0) indicating no activity; (1) minimal activity; (2) mild activity; (3) moderate and (4) severe activity. IHC was performed on colon tissue from 11 non-inflamed UC patients and 25 patients with varying degrees of inflammation. Data represents mean ± SEM; **p* < 0.05; ***p* < 0.01, ****p* < 0.001 (Mann Whitney U test).

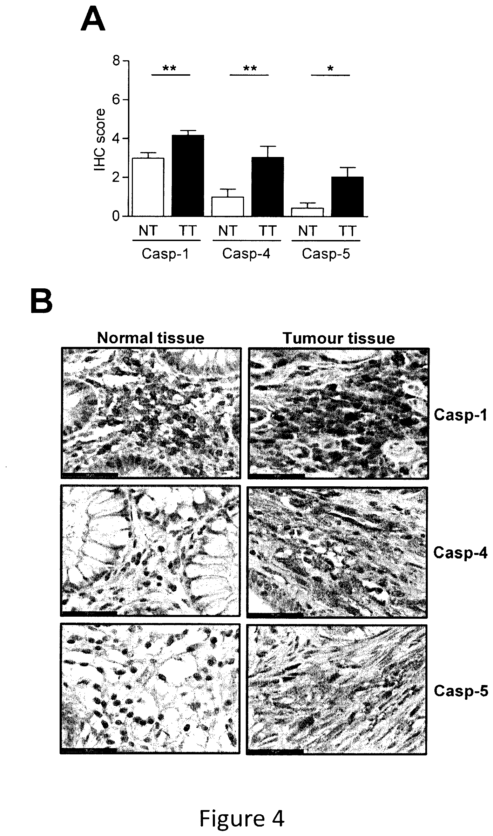

**Figure 4****: Quantitative analysis of IHC staining reveals increased caspase-1, -4 and -5 expression in stromal cells of CRC tumour tissue**
   (A) Defined areas of tumor tissue (TT) and adjacent normal tissue (NT) from CRC patient resections were IHC scored for caspase-1, -4, and -5 expression in stromal cells. (B) Representative images of caspase-1, -4 and -5 expression patterns in stromal cells from normal and tumor CRC patient resection tissue. Data represent mean ± SEM; **p* < 0.05, ***p* < 0.01, ****p* < 0.001 (Mann Whitney U test). Caspase-1 NT (n=18), caspase-1 TT (n=22); caspase-4 NT (n=18), caspase-4 TT (n=23); and caspase-5 NT (n=18), caspase-5 TT (n=24). Scale bar = 50 µm.
**Figure 5****: Epithelial expression of caspase-4 and -5 is both specific to, and restricted to, neoplastic CRC tumor tissue**
   (A) Defined areas of tumor tissue (TT) and adjacent normal tissue (NT) from CRC patient resections were IHC scored for caspase-1, -4, and -5 expression in epithelial cells. (B) Representative images of caspase-1, -4 and -5 expression patterns in epithelial cells from normal and tumor CRC patient resection tissue. Data represent mean ± SEM; **p* < 0.05, ***p* < 0.01, ****p* < 0.001 (Mann Whitney U test). Caspase-1 NT (n=18), caspase-1 TT (n=22); caspase-4 NT (n=18), caspase-4 TT (n=23); and caspase-5 NT (n=18), caspase-5 TT (n=24). Scale bar = 50 µm.

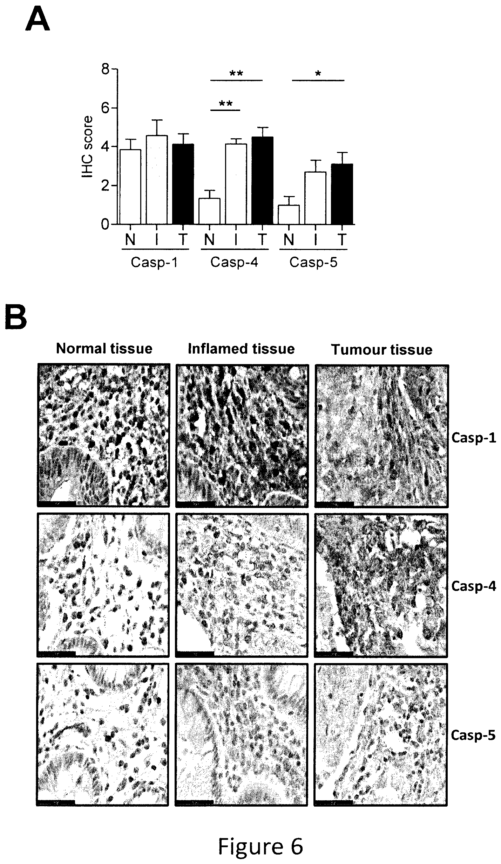

**Figure 6****: Increased stromal expression of inflammatory caspase-4 and -5 in inflamed and tumour sections of colitis associated CRC tissue**
   (A) Defined areas of: (N) normal (n=6); (I) inflamed (n=7); and (T) tumor (n=8) tissue from CAC patient biopsies were scored for caspase-1, -4 and -5 expression in stromal cells. (B) Representative images of caspase-1, -4 and -5 expression patterns in normal, inflamed and tumor stromal tissue from CAC patient biopsies. Data represent mean ± SEM; **p* < 0.05; **p* < 0.01 (Mann Whitney U test); Scale bar = 50 µm.

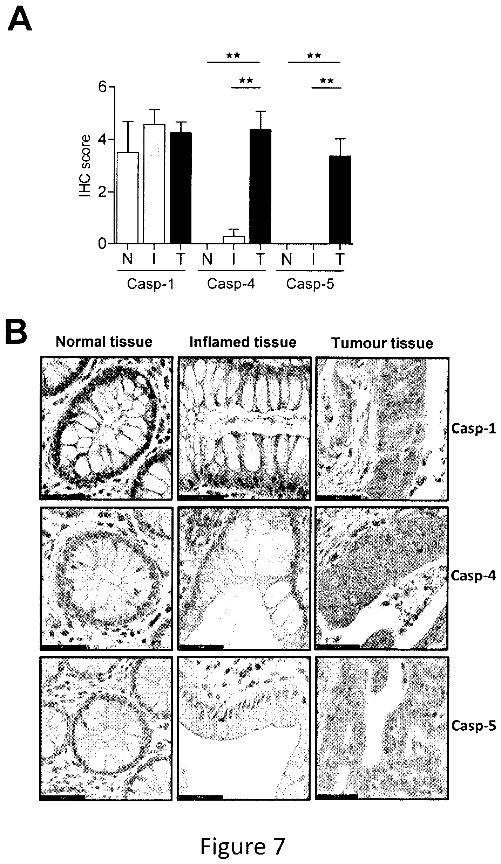

**Figure 7****: IHC staining of biopsies from CAC (colitis associated CRC) patient resections confirm tumor-specific epithelial expression of caspases-4 and -5.**
   (A) Defined areas of: (N) normal (n=6); (I) inflamed (n=7); and (T) tumor (n=8) tissue from CAC patient biopsies were scored for caspase-1, -4 and -5 expression in epithelial cells. (B) Representative images of caspase-1, -4 and -5 expression patterns in normal, inflamed and tumor epithelial tissue from CAC patient biopsies. Data represent mean ± SEM; **p* < 0.05; **p* < 0.01 (Mann Whitney U test); Scale bar = 50 µm.

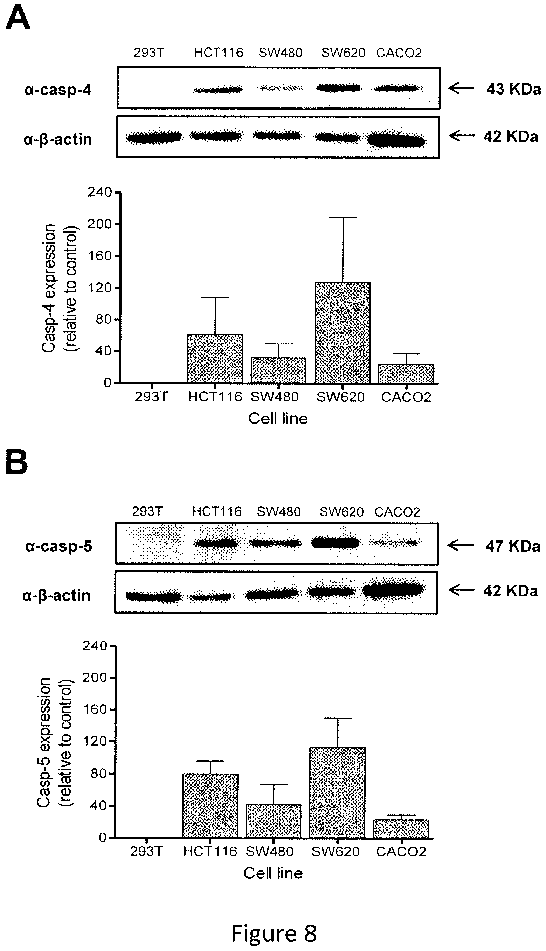

**Figure 8****: Increased expression of caspase-4 and -5 in a panel of CRC cell lines**
   Western blot and subsequent densitometry was performed probing for caspase-4 and caspase-5 in lysates from 293T (control), HCT116, SW480, SW620 and CACO2 cells. Proteins (20 µg) were fractionated by SDS-PAGE and detected by Western Blot analysis using anti-caspase-4 (A) and anti-caspase-5 (B). Expression levels were normalised to β-actin to account for loading issues. Results are representative of three independent experiments.

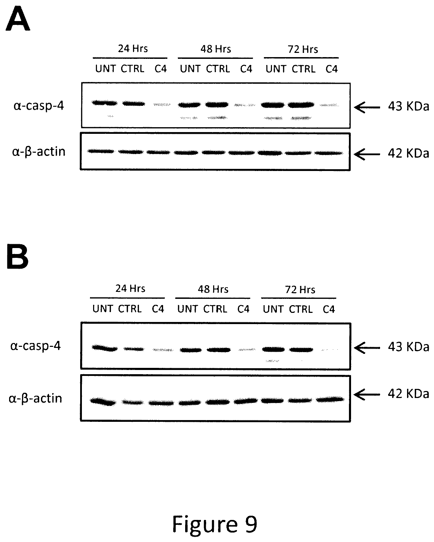

**Figure 9****: Inhibition of caspase-4 expression after transfection with siRNA.**
   Western blot analysis of (A) Caco-2 and (B) SW620 cells treated with 100 mM siCASP4 for 24, 48 and 72 hours. Proteins (20 µg) were fractionated by SDS-PAGE and detected by Western Blot analysis using anti-caspase-4. β-actin was included to assess equal protein loading.

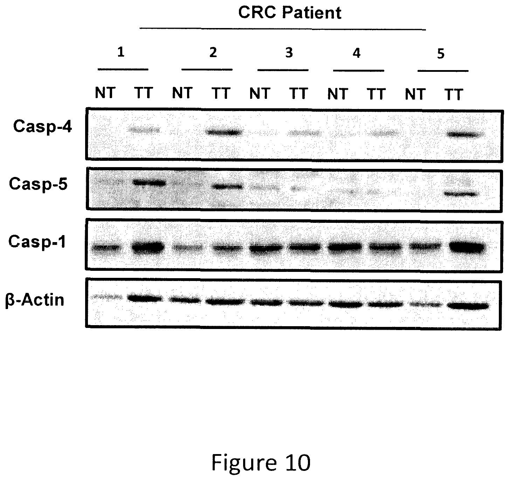

**Figure 10****: Increased caspase-4 and -5 protein levels in tumour tissue from CRC patients are detectable by Western blot.**
   Distal to tumour (non-adjacent) normal (N) and tumour tissue (T) lysates (20 µg) from CRC patient resections were probed by Western blot for caspase-1, -4 and -5.

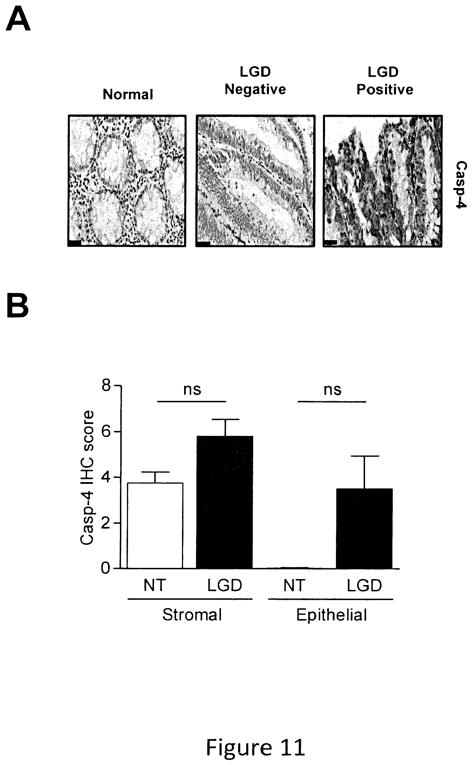

**Figure 11****: IHC staining of Polyp tissue confirms tumour-specific epithelial expression of caspases-4 and -5.** (A) Representative images of caspase-4 expression in defined areas of normal (N) and low grade dysplastic (LGD) tissue from colon polyp tissue. (B) Defined areas of N and LGD from colon polyp tissues (n=5) were IHC scored for stromal and epithelial caspase-4 expression. Data represent mean ± SEM; **p* < 0.05; **p* < 0.01 (Mann Whitney U test); Scale bar = 50 µm.

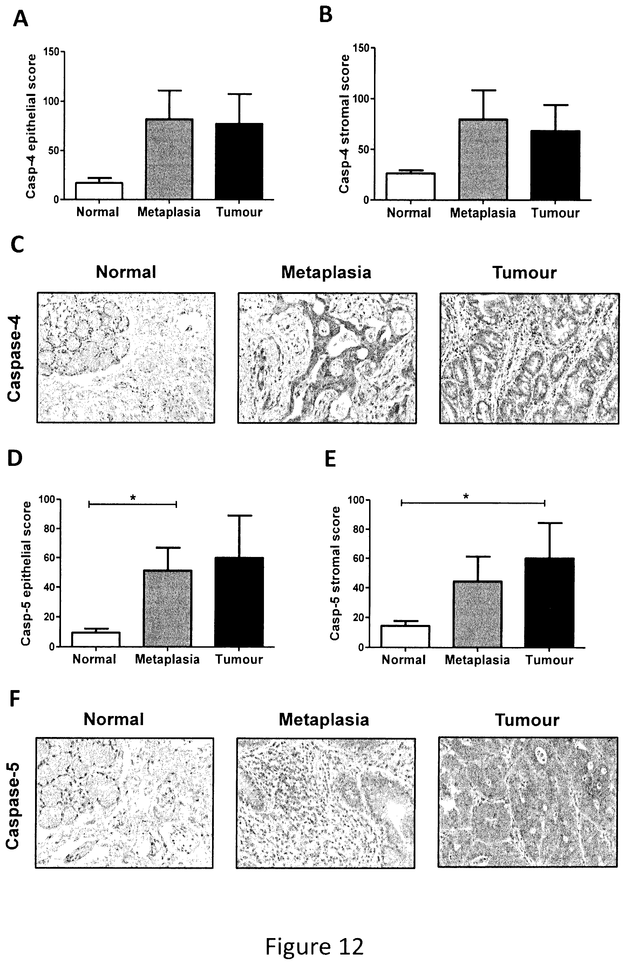

**Figure 12****: IHC stained Oesophageal tissue from Healthy, Barrett's (Inflamed) and adenocarcinoma (OAC) patients reveal increased and distinct caspase-4 and -5 staining in metaplastic and tumour regions.** IHC scores from defined areas of adjacent normal tissue, intestinal metaplasia (Barrett's oesophagus) and tumour tissue from oesophageal adenocarcinoma patient resections were IHC scored for (A and B) caspase -4; and (D and E) caspase-5 expression in epithelial and stromal cells. (C and F) Representative images of caspase-4 and -5 expression patterns in epithelial and stromal cells from adjacent normal, Barrett's oesophagus and esophageal adenocarcinoma tumour patient resection tissue. Data represent mean ± SEM; **p* < 0.05, (Mann Whitney U test). Caspase-4 (n=5), caspase-5 (n=5). Original magnification ×10.

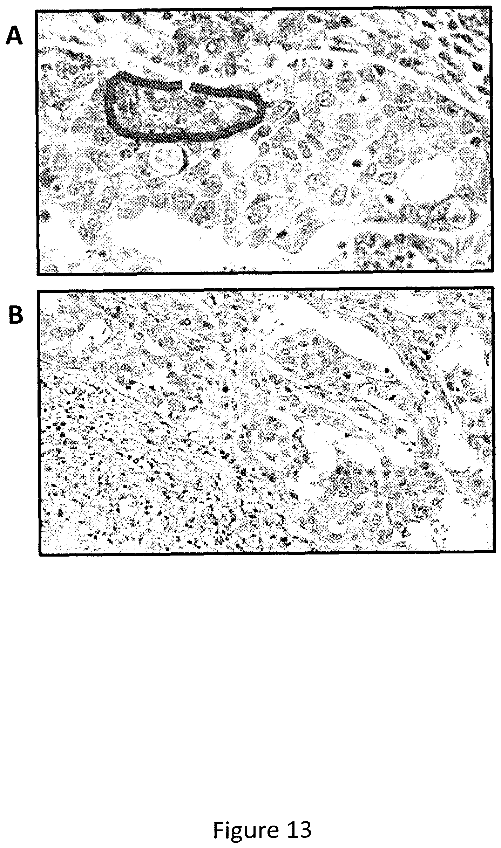

**Figure 13****: IHC stained breast carcinoma tissue reveals tumour-specific epithelial positivity for caspase-4.** Representative images showing tumour-specific epithelial caspase-4 expression. (A) 40X magnification of breast tumour area (green) with positive staining for caspase-4 (red). (B) 20X magnification of breast tumour tissue from another patient showing caspase-4 positivity in tumour epithelial cells.

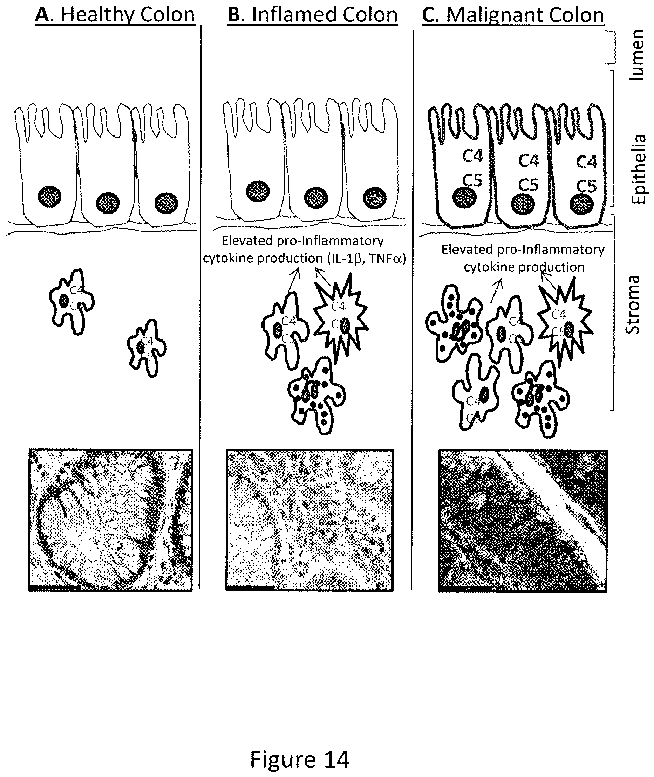

**Figure 14****: Schematic representation of caspase-4 and -5 expression levels in normal and diseased colon. A.** In colons from healthy individuals, epithelial cells are completely negative for caspase-4 and -5 expression, and low background levels of caspase-4 and -5 (C4, C5) are detected within the stroma. **B.** Inflamed colons from ulcerative colitis patients display increasing expression levels of caspases-4 and -5 in the stroma, which correlate with levels of inflammation and disease activity. Epithelial cells from inflamed tissue are still negative for caspase-4 and -5 expression. **C.** Malignant tissue from patients with colorectal cancer are positive for caspase-4 and -5 expression in both epithelial cells and within the stroma. Images represent patient biopsy tissue that has been immunohistochemically stained for caspase-4 expression in (A) healthy, (B) inflamed and (C) malignant colon tissue.

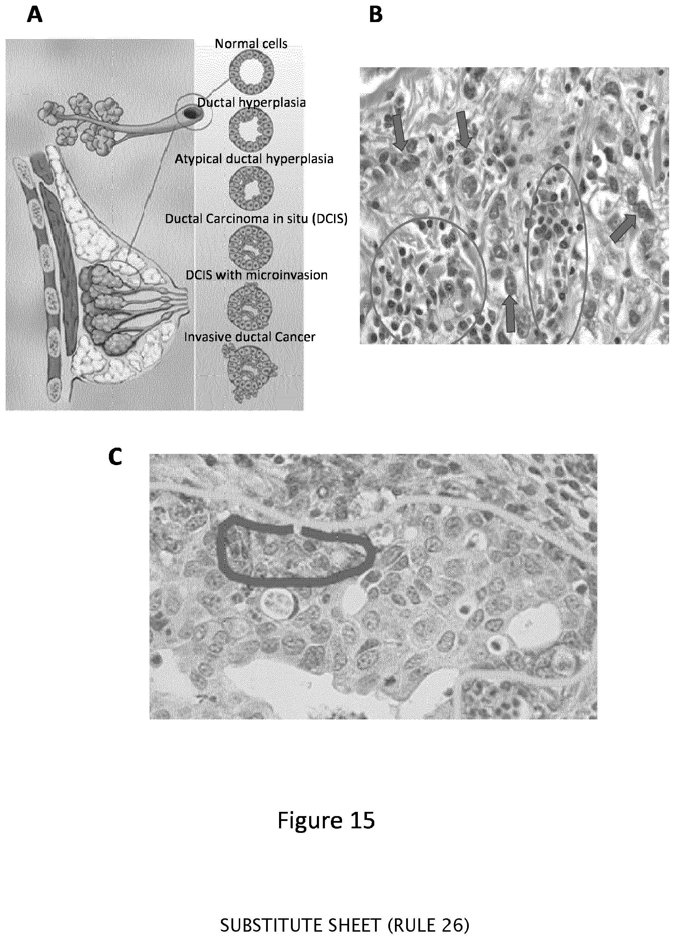

**Figure 15****: Schematic representation of caspase-4 and -5 expression levels in breast adenocarcinoma. A.** Schematic representation of ductal carcinoma in situ and local invasion. Breast adenocarcinomas are derived from the epithelium lining the ducts or lobules. **B.** Representative image showing H&E staining of breast adenocarcinoma tissue. Malignant cells are represented by the red arrows. Intermixed inflammatory cells are highlighted by the blue circles. **C.** Representative image from adenocarcinoma tissue that has been immunohistochemically stained for caspase-4 expression. Green line represents tumour boundary, red area highlights caspase-4 positive epithelial tumour cells.

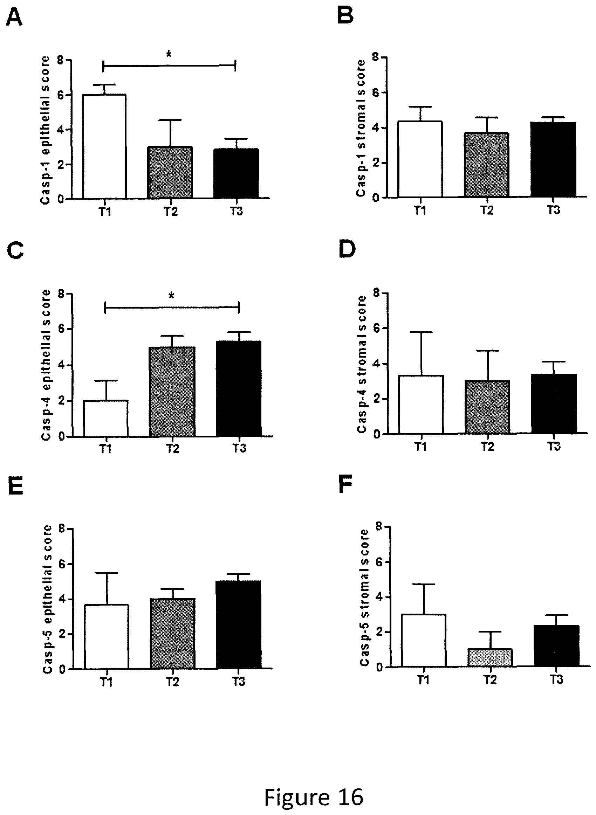

**Figure 16****: Inflammatory caspase expression profile across tumour grades** - **Increased epithelial caspase-4 expression in advanced tumour grades.** IHC scores for caspase-1, -4 and - 5 expression in epithelial (A, C and E) and stromal (B, D and F) areas of tumour tissue from CRC patients (n=20) were correlated to their tumour grade. Tumour grades (T1 (tumour is confined to the inner layer of the bowel), n=3; T2 (tumour has advanced into the muscle layer of the bowel wall), n=3 and T3 (tumour has grown into the outer lining of the bowel wall), n=14) were assigned by a pathologist using the TNM (Tumour-Node-Metastasis clinical classification system). Data represents mean ± SEM; two tailed independent student *t*-test; **p* < 0.05.

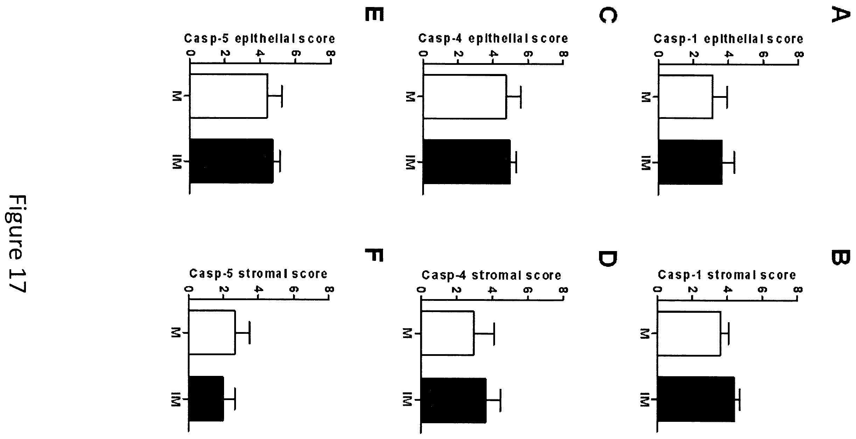

**Figure 17****: No significant differences between Inflammatory Caspase Expression and CRC 'Moderate' or 'Invasive Moderate' differentiation status.** IHC scores for caspase-1, -4 and -5 expression in epithelial (A, C and E) and stromal (B, D and F) compartments of tumour tissue from CRC patients (n=21) were correlated to their differentiation status: M (moderately differentiated adenocarcinoma, n=9) and IM (moderately invasive differentiated adenocarcinoma, n=12). Data represents mean ± SEM; two tailed independent student t-test.

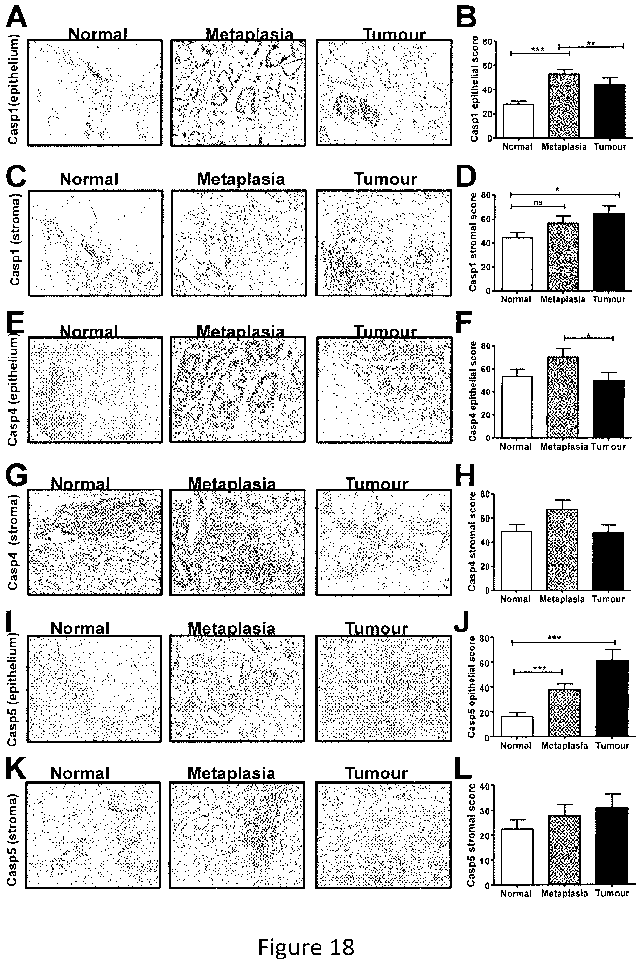

**Figure 18****: Increased caspase-1, -4 and -5 expression in areas of inflammation (metaplasia) and tumour in oesophagectomy tissue from adenocarcinoma (OAC) patients.**
   Representative images of caspase-1 ,-4 and-5 expression in epithelial cells (A, E, I) and stromal cells (C,G,K) from within defined areas of adjacent normal, Barrett's oesophagus (metaplasia) and oesophageal adenocarcinoma (tumour) patient resection tissue. Defined areas of adjacent normal, intestinal metaplasia and tumour tissue from OAC patients were IHC scored for caspase-1,-4 and -5 expression in (B,F,J) epithelial and (D,H,L) stromal regions, using a validated semi-quantitative scoring method (n=32 patients per group). Data represent mean ± SEM. p values were calculated using the Mann-Whitney *U*-test; **p* < 0.05, ***p* < 0.01, ***p<0.001 and ns (not significant). Original magnification ×20.

### EXAMPLES

### EXAMPLE 1 - INFLAMMATORY BOWEL DISEASE AND ADENOCARCINOMA

**Design** Inflammatory caspase expression was examined in IBD patients from a Belgian cohort by qPCR (n=103); and in ulcerative colitis (UC) patients (n=36), CRC patients (n=25) and UC-associated CRC patients (n=8) from Irish cohorts by immunohistochemical (IHC) staining.

### METHODS

### Clinical samples

*Belgian IBD Patient population:* This study was approved by the regional ethics committee (EC 2000/242), and all participating patients signed informed consents. In total, 103 biopsies were retrieved from controls (n=23; 10 males, 13 females, average age 51 (range 22-69)), and patients with CD (n=54; 27 males, 27 females, average age 37 (range 8-72)) or UC (n=16; 8 males, 8 females, average age 38 (range 7-61)). All patients within this cohort were medication free or on 5-ASA. In addition, the majority of patients have previously been included in a genome-wide scan. *Irish UC Patient population:* This study was approved by the Research and Ethics committee of St. Vincent's University Hospital. All subjects provided written informed consent. Intestinal biopsies were retrieved from 36 patients with a diagnosis of UC, with prospective Mayo scores of endoscopic activity: Mayo 0 (inactive, n=11; 3 males, 8 females, average age 46 (range 19-60)), Mayo 1 (minimal, n=7; 6 males, 1 female, average age 41 (range 34-64)), Mayo 2 (mild, n=8; 8 males, average age 37 (range 22-76)), Mayo 3 (moderate, n=4; 4 males, average age 49 (range 38-50)) and Mayo 4 (severe, n=6; 3 males, 3 females, average age 31 (range 20-72)). All but 4 patients in this cohort were receiving medication including aminosalicylates, corticosteroids, immunomodulators or biological therapy (adalimumab or infliximab) according to best clinical practice for treatment of their disease.

*Irish CRC and IBD-associated CRC patient population:* The protocol was approved by the Medical Research Ethics Committee, Beaumont hospital. Areas of tumor and adjacent normal tissue from 25 spontaneous CRC patients (see Table 1 for characteristics) were assessed by IHC for inflammatory caspase-1, -4 and -5 expression levels. Explant tissue from a further 8 patients with IBD-associated CRC (see Table 2 for characteristics) were assessed for inflammatory caspase expression in areas of tumor, inflammation and normal adjacent tissue.

**Table 1**

| **Patient No.** | **Gender** | **Age** | **Location** | **Type** | **Stage** |
|---|---|---|---|---|---|
| **1** | F | 76 | Right hemicolectomy | Invasive moderately differentiated adenocarcinoma | pT3 N2 Mx Duke's C |
| **2** | M | 51 | Anterior resection | Moderately differentiated adenocarcinoma | pT3 N1 Mx Duke's C |
| **3** | F | 78 | Right hemicolectomy | Invasive moderately differentiated adenocarcinoma | pT3 N0 Mx Duke's B |
| **4** | F | 71 | Anterior resection | Invasive moderately differentiated adenocarcinoma | pT4 N2 Mx Duke's C |
| **5** | M | 78 | Right hemicolectomy | Villius adenomata | pT is N0 Mx |
| **6** | F | 41 | Anterior resection | Focally invasive moderately differentiated adenocarcinoma, post neo-adjuvant chemotherapy | ypT3 N0 Duke's B |
| **7** | M | 61 | Sigmoid | Invasive moderately differentiated adenocarcinoma | pT3 N0 Mx Duke's B |
| **8** | F | 78 | Right hemicolectomy | Invasive moderately differentiated adenocarcinoma | pT2 N0 Mx Duke's A |
| **9** | M | 64 | Rectum | Invasive moderately differentiated adenocarcinoma | pT2 N2 Mx Duke's C |
| **10** | M | 59 | Rectum | Invasive moderately differentiated adenocarcinoma, post neo-adjuvant chemotherapy | ypT3 N0 Duke's B |
| **11** | F | 50 | Right hemicolectomy | Ulcerated moderately differentiated adenocarcinoma | pT3 N0 Mx |
| **12** | M | 74 | Sigmoid | Moderately differentiated adenocarcinoma | pT3 N1 Mx Duke's C |
| **13** | F | 62 | Anterior resection | Infiltrating moderately differentiated adenocarcinoma | pT1 N0 Duke's A |
| **14** | F | 44 | Recto-sigmoid | Moderately differentiated adenocarcinoma | pT2 N0 Mx Duke's A |
| **15** | F | 39 | Proctocolectomy | Synchronous adenocarcinoma in FAP | pT3 N1 Mx Duke's C |
| **16** | M | 64 | Right hemicolectomy | Invasive moderately differentiated mucinous adenocarcinoma | pT3 N2 Mx Duke's C |
| **17** | F | 53 | Right hemicolectomy | Invasive moderately differentiated adenocarcinoma | pT3 N0 Mx Duke's B |
| **18** | F | 65 | Right hemicolectomy | Invasive moderately differentiated adenocarcinoma | pT3 N0 M1 |
| **19** | F | 72 | Sigmoid | Invasive moderately differentiated adenocarcinoma | pT1 N0 Mx |
| **20** | F | 81 | Rectum | Moderately differentiated adenocarcinoma | pT1 N0 Mx |
| **21** | F | 69 | Right hemicolectomy | Moderately differentiated adenocarcinoma | pT3 N1 Mx Duke's C |
| **22** | F | 78 | Colon (no area specificed) | Ulcerated moderately differenciated adenocarcinoma | pT4 N0 |
| **23** | F | 81 | Colon (no area specificed) | Well differentiated adenocarcinoma | pT3 N1 Mx Duke's C |
| **24** | M | 83 | Anterior resection | Moderately differentiated adenocarcinoma | pT3 N2 |
| **25** | M | 70 | Recto-sigmoid | Poorly differentiated adenocarcinoma, post neo-adjuvant chemotherapy | ypT3 N0 Mx |

**Table 2**

| **Patient No.** | **Gender** | **Age** | **Tumor Type** | **Stage** |
|---|---|---|---|---|
| **1** | F | 76 | Invasive poorly differentiated adenocarcinoma | pT4 N2 |
| **2** | M | 56 | Ulcerated mucinous moderately differentiated adenocarcinoma | pT4 N2 |
| **3** | M | 49 | Moderately differentiated adenocarcinoma | pT1 N1, Duke's C |
| **4** | F | 66 | Well to moderately differentiated adenocarcinoma, had neoadjuvant chemo | ypT3 N0 |
| **5** | M | 42 | Invasive signet ring cell adenocarcinoma | pT3 N1 Mx |
| **6** | M | 70 | Poorly differentiated adenocarcinoma | pT3 N2 |
| **7** | M | 36 | Focally invasive moderately differentiated adenocarcinoma | Duke's A |
| **8** | M | 44 | Moderately differentiated mucinous adenocarcinoma | pT3 N0 Duke's B |

### RNA isolation and conversion to cDNA

Total RNA was extracted from biopsies using the Qiagen RNeasy Mini Kit, with on-column DNAse treatment according to manufacturer's instructions. Quality and concentration of RNA was assessed by subjecting samples to automated gel electrophoresis and Experion analysis (Bio-Rad). Samples with a 28S/18S ratio between 1.6 and 1.8 and a minimum RNA Quality Index of 7 included in the analysis. The WT-Ovation™ system from NuGEN was used to prepare and amplify cDNA, starting from 50 ng of total RNA, according to the manufacturer's instructions.

### Quantitative PCR reaction

*Primer design:* RTprimerDB was used for primer design and *in silico* primer evaluation (absence of secondary structures, SNPs and aspecific binding by means of BLAST). The conditions of qPCR were set to 60°C annealing temperature, 50 mM Na⁺ and 3 mM Mg²⁺. To test the PCR-efficiency of a primer set, a dilution series of cDNA or gDNA were subjected to qPCR analysis, and the efficiency was calculated as (10^{-1/slope} - 1) x 100. Primer sets with efficiencies of 90-110% were used. Primer sequences used were as follows:
CASP1F-TGCCTGTTCCTGTGATGTGGAGGA; CASP1R-CAGTGGTGGGCATCTGCGCT;
CASP4F-ACCGTGGGGAACTGTGGGTCA; CASP4R- CCAGGACACGTTGTGTGGCGT;
CASP5F-CGCAGACGCCTGGCTCTCAT; CASP5R- AGCCCAGGCCTTGAAGCAGC.

SYBR green I containing PCR mixtures (Roche, 8 µl reaction volume) was run on a Lightcycler in 384 well format (LC480, Roche). Cycling conditions were 10 min 95°C and 40 cycles at 95°C for 15 s and 60°C for 60 s. For each primer set, melting-peak analysis was performed to verify the specificity of the reaction in each well. Expression data were normalized using the median expression of SDHA, HPRT and GAPDH as reference genes. Data analyses were performed using the accompanied software and results were imported to Prism (Graphpad software) for further analysis.

### Validation of the cDNA collection using interleukin 8 (IL-8)

It is to be expected that IL-8 levels in macroscopically inflamed biopsies are elevated as compared to tissue obtained from healthy control individuals. However, it cannot be excluded that unaffected biopsies isolated from IBD patients will be completely devoid of any microscopic inflammation. Therefore, IL-8 levels of all samples were measured by qPCR. A high correlation was found between endoscopic (macroscopic) evaluation of inflammation and IL8 levels, with a specificity of 81% and a sensitivity of 72%.

### Immunohistochemistry of UC Patient biopsies

Immunohistochemistry was performed using Formalin-fixed, paraffin-embedded (FFPE) tissues obtained from UC patients. A routine three-stage immunoperoxidase labelling technique incorporating avidin-biotin immunoperoxidase complex (DAKO, Glostrup, Denmark) was used. Sections were incubated with primary anti-caspase-1 (Santa Cruz Biotech.), anti-caspase-4 and -5 (Abcam/MBL) antibodies for 1 h. Sections were also incubated with an appropriate isotype matched mouse/rabbit monoclonal antibody as a negative control. DAB, diaminobenzadine tetrahydrochloride (Sigma) was used to visualise staining. Images were captured using Olympus DP50 light microscope and AnalySIS software (Soft Imaging System Corporation, Lakewood, Colorado, USA). Caspase expression was assessed by two blinded reviewers using a validated semi-quantitative scoring method. All IHC stained cells were assessed by a combined score of intensity and percentage of nuclear and cytoplasmic staining. Intensity of staining was graded using a scale of 0 - 3 where 0 = negative, 1 = weak, 2 = moderate and 3 = strong. Percentage positivity was graded using a scale of 0 - 4 where 0 = no stained cells, 1 = 1 - 25% stained cells, 2 = 25 - 50% stained cells, 3 =50 - 75% stained cells and 4 = 75 - 100% stained cells.

### CRC immunohistochemical analysis

Inflammatory caspase IHC analysis was carried out on a BOND-III immunostainer from Leica Biosystems, Newcastle, UK. The Bond-III system dewaxed slides before pre-treatment with BOND Epitope Retrieval Solution I (Cat. no. AR9961). Primary antibodies were diluted in Bond Primary Antibody Diluent (Cat. no. AR9352) - anti-Casp-4 (Cat. no. M029-3; MBL); anti-Casp-5 (Cat. no. M060-3; MBL) and anti-Casp-1 (Cat. no. SC 622; Santa Cruz Biotechnology). Detection and visualisation of stained cells was achieved using the Bond Polymer Refine Detection Kit (Cat. no. DS9800), using diaminobenzidine (DAB) as the chromagen. Tissues were counterstained with haematoxylin and cover slipped. Appropriate negative controls (omission of primary antibodies) were used in all assays.

### Statistical analysis

Values are expressed as the median and range or mean ± SEM. The software package GraphPad Prism, version 5 (GraphPad Software, CA, USA) was used to perform statistical analysis. Differences in parameters between two groups were performed using the non-parametric Kruskal-Wallis test (with Dunn's post hoc comparison to control) for qPCR analysis and unpaired Mann-Whitney tests for IHC expression data. p values of <0.05 were considered statistically significant. Correlation analysis was undertaken by calculating the spearman correlation coefficient. A p value of less than 0.05 was considered significant.

### RESULTS

### Elevated expression levels of inflammatory caspases in inflamed IBD patient samples

A study was designed to examine the extent of inflammatory caspase expression in IBD patient intestinal biopsies. We used real time PCR to amplify and quantify *CASP1, CASP4* and CASP5 from cDNA prepared from 103 biopsies of 53 CD patients, 26 UC patients and 24 healthy patients. UC/CD healthy or inflamed tissue was obtained from UC/CD patients with and without endoscopic evidence of inflammation and control tissue was obtained from endoscopically normal biopsies from non-IBD control individuals. Caspase-1 expression was significantly elevated in tissue samples from inflamed UC/CD patients (figure 1A). Both caspase-4 and caspase-5 expression was also significantly elevated in inflamed tissue, suggesting that, in addition to caspase-1, caspases-4 and -5 may also have a role during intestinal inflammation (figure 1B, C). The data shown in Figure 1C revealed that caspase-4 expression is higher in non-inflamed tissue from IBD patients (in both ileum and colon), suggesting that it may be an early marker of IBD. In contrast, both caspase-1 and -5 expression was significantly higher in inflamed colonic, not ileal, tissue (figure 1A, C), suggesting their involvement with colitis, rather than ileitis. Taken together, these data support the hypothesis that inflammatory caspases-4 and -5 have an important role in intestinal inflammation observed during IBD.

### Stromal caspase-4 and -5 expression levels correlate with pathological inflammation and clinical disease scores in UC patients

To confirm this hypothesis, we examined the relationship between inflammatory caspase expression and both inflammation and disease activity in UC patients. Disease activity (Mayo) scores were prospectively assigned to IBD patients by the physician at the time of endoscopy, and biopsies from 36 UC patients with Mayo scores ranging from 0 to 4 (i.e. normal/inactive disease to severe disease) were coded and sectioned for histological assessment, to assign an inflammatory score; and immunohistochemical (IHC) assessment, to stain for caspase-1, -4 and -5 expression. The data demonstrate a STRONG correlation between the clinical Mayo scores and the histological inflammation scores (SPEARMAN'S RHO 0.631, P<0.0001) (Figure 2A), confirming the validity of both scoring systems. The data shown in figure 2B revealed that, although caspase-1 appears to be expressed at low levels in non-inflamed tissue, it becomes strongly expressed in both epithelial cells and infiltrating immune cells within the lamina propria during inflammation. A comparison of caspase-1 IHC scores with the inflammation scores reveals that caspase-1 appears to be maximally expressed once inflammation begins and is sustained during moderate and strong inflammation (figure 2C). Similarly, increased caspase-1 staining also WEAKLY correlated with the endoscopic Mayo score up to a moderate level (score of 3), (figure 2D). Caspase-4 and -5 expression patterns were very similar, consistent with their previously reported tissue distribution levels and their 74% sequence identity, and were restricted to infiltrating immune cells within the lamina propria (figure 3A, D). In contrast to caspase-1, caspases-4 and -5 were not detectable within the epithelial cells of patients with UC, regardless of the degree of inflammation (figure 3A, D). Expression of caspase-4 in stromal tissue correlated very clearly with the extent of inflammation (SPEARMAN'S RHO 0.502, P<0.0003) (figure 3B), with maximum caspase-4 expression present in sections with inflammatory scores from moderate to strong (scores of 2 and 3); and also CORRELATING STRONGLY disease activity up to a moderate level (SPEARMAN'S RHO 0.622, P< 0.0006) (figure 3C). Similarly, stromal caspase-5 IHC staining intensity and percentage correlated STRONGLY with increasing DISEASE ACTIVITY scores (SPEARMAN'S RHO 0.713, P<0.0001) (figure 3E) and increasing INFLAMMATION SCORES up to a moderate level (SPEARMAN'S RHO 0.422, P<0.0038) (figure 3F). Thus, data from this study reveal a clear involvement for caspases-4 and -5 in UC-associated inflammation, and represent novel targets for the management of intestinal inflammation in IBD patients.

### Expression of caspases-4 and -5 in epithelial cells from tumor, but not adjacent-normal, tissue from CRC patients

IBD is a risk factor for the development of colorectal cancer (CRC). Having demonstrated the involvement of inflammatory caspases during IBD, we sought to determine whether their expression was also elevated in CRC. Colorectal tumor and adjacent-normal tissue samples from 25 CRC patients were stained for expression of caspase-1, -4 and -5 by IHC. Each of the CRC samples, regardless of the stage of tumor progression or pathway of genetic instability (table 1), exhibited similar profiles for each of the inflammatory caspases. Within the stromal cell layers, caspase-1 was expressed in infiltrating immune cells of tumor tissue, and to a lesser extent, in those of adjacent normal tissue. Interestingly, there was a more marked increase in the stromal expression of caspase-4 and -5 between tumor tissue and adjacent normal tissue, probably reflecting the enhanced inflammation occurring within the local tumor microenvironment (figure 4A, B). Inflammatory caspase expression in epithelial cell layers contrasted greatly to the profiles observed in the stroma (figure 5A, B). Epithelial cell layers exhibited a striking difference in caspase-4 and -5 expression levels between normal and neoplastic tissue. Similar to our observations from UC patient biopsies, no expression of either caspase-4 or -5 could be detected in adjacent normal tissue, however high levels of caspase-4 and -5 are present in tumor epithelial cells (figure 5A, B). In contrast, caspase-1 expression appears to be down-regulated in epithelial carcinoma cells (figure 5A, B), which agrees with previous findings[10]. This data suggests that caspases-4 and -5 may be novel markers of colon carcinoma.

Figure 16 and 17 are part of the same study as the results shown in Figures 4 and 5. They are from the same cohort of patients, but the information has been further dissected into tumour grade (T1-T3) and differentiation status (M/IM) of patients within that cohort.

Figures 16 and 17: We carried out further analysis on the caspase expression levels within the same cohort of patients. This further analysis of caspase expression levels within each of the tumour grades (T1-T3) confirms that epithelial caspase-1 expression appears to decrease with increasing tumour grade (Figure 16A), whereas epithelial caspase-4 levels appear to increase with increasing tumour grade (Figure 16C), suggesting that determining caspase-1 and -4 levels within CRC patients may be indicative of tumour grade. In contrast, caspase-5 levels appear to be robustly elevated even at T1, the earliest tumour stage, when tumour growth has not spread beyond the inner layer of the colon (Figure 16E). This is a salient observation, as it suggests that caspase-5 may be more relevant than caspase-4 in terms of early stage tumour detection.

Caspase expression levels were also examined according to the differentiation status of the malignancies. Moderately differentiated adenocarcinomas, which are usually present in colorectal cancers, represented the majority of tumours within this cohort. These were further categorized into moderate (M) or invasive-moderately (IM) differentiated by a clinical pathologist. Analysis of caspase expression levels in both epithelial and stromal cell layers revealed no significant differences between either differentiation status (Figure 17). This observation suggests that inflammatory caspase expression levels do not influence the invasiveness of CRC malignancies.

### Expression of caspases-4 and -5 in epithelial cells from tumor, but not adjacent-normal or inflamed, tissue from UC-associated CRC patients

Current chemoprevention strategies for IBD patients include maintenance anti-inflammatory medications, to prevent dysplasia/cancer development; and surveillance colonoscopy, to identify and treat early lesions. However, dysplastic lesions associated with IBD-CRC are often difficult to detect and to grade for severity[11]. To determine at which point in the inflammation-dysplasia-carcinoma sequence expression of caspase-4 and -5 in epithelial cells is upregulated, a set of UC-associated CRC patient resections were examined in areas of normal, inflamed and tumor tissue for inflammatory caspase expression. Stromal tissue from these patients had similar expression profiles to those previously observed in UC patients (figure 3) and CRC patients (figure 4A, B); with increased expression of caspases-4 and -5 in both inflamed and tumor tissue, while normal tissue displayed high levels of caspase-1, making it less specific to the inflammatory/malignant status of the tissue (figure 6A, B). Strikingly, epithelial expression of caspases-4 and -5 were still dramatically restricted to neoplastic tissue, even within areas of severely inflamed tissue (figure 7A, B). Areas of normal, inflamed and dysplastic tissue from CRC patient resections all remained completely negative for epithelial caspase-4 and -5 expression (figure 7A, B), although a marked increase in stromal and intraepithelial infiltrating lymphocytes and macrophages was observed in areas of dysplastic tissue.

In support of these findings, a panel of CRC cell lines were also all found to be positive for the expression of caspases-4 and -5 (Figure 8a, b). Experiments to specifically silence caspases-4 and - 5, using siRNA, aim to determine the functional implications of their expression during malignancy (figure 9). These findings reveal that caspases-4 and -5 are novel discriminators between inflamed and neoplastic tissue, and suggest that there may be significant scope for the detection of epithelial caspase-4 and -5 as determinants of malignancy within colorectal mucosa. The presence of caspase-4 and -5 in epithelial cells could be developed as a complimentary marker to histological assessment of dysplasia within colon tissue when predicting CRC risk in patients.

### DISCUSSION

This study demonstrates that inflammatory caspases are highly expressed by infiltrating immune cells in areas of active inflammation in the gastrointestinal tract of IBD patients. Relative expression levels, particularly of caspases-4 and -5, correlate very closely with the level of tissue inflammation and the grade of disease activity in UC patients, suggesting that their enhanced expression has a significant role in disease pathogenesis. Similarly, we demonstrated that there were high expression levels of caspase-4 and -5 in the stromal compartment of colorectal cancer. Strikingly, we found a dramatic switch in the expression pattern of caspase-4 and -5 in epithelial cells, from no expression in normal or inflamed tissues, to strong expression in neoplastic tissue.

The expression of inflammatory caspases within the stroma of both IBD patients and CRC patients appears to be primarily within infiltrating immune cells, with macrophages, lymphocytes and plasma cells all found to express caspase-4 and -5. A strong correlation between inflammation score and caspase-4 and -5 expression was observed in UC patient biopsies, which supports the hypothesis that caspases-4 and -5 contribute to intestinal inflammation, most probably through activation of the non-canonical inflammasome, leading to increased IL-1beta and IL-18 production. Although evidence for the importance of caspase-4/-5 mediated non-canonical inflammasome activation during human inflammation is emerging [12], the majority of studies to date characterizing non-canonical inflammasome activation have been carried out in mice. Our novel findings provide evidence of caspase-4 and -5 mediated non-canonical inflammasome activation during intestinal inflammation in UC patients.

Inflammation is an essential driving force in the development of epithelial-originated tumors, and our observation of an increased infiltration of caspase-4 and -5 expressing immune cells to the developing tumor site may serve to establish the tumor inflammatory microenvironment[]. Instead of repressing tumor growth, inflammatory cells can promote tumor growth, particularly during inflammation-associated cancer. Another significant observation from this study is the strong expression of caspases-4 and -5 within the neoplastic epithelium of CRC patients. Within tumor areas, stromal staining for caspases-4 and -5 was even stronger than that observed in inflamed tissue, and epithelial cells became strongly positive for caspase-4 and -5, suggesting that inflammatory cells may have a role in inducing the expression of caspase-4 and -5 in colon carcinomas. Exposure of epithelial cells, or more likely their precursor intestinal stem cells, to pro-inflammatory cytokines can induce cancer stem cell (CSC) markers and cause tumor formation *in vivo.* This may be a plausible mechanism for the switch to caspase-4 and -5 expression in neoplastic epithelial cells. High levels of infiltrating immune cells displaying caspase-4 and -5 mediated inflammasome activation, which is driving the production of pro-inflammatory cytokines, may facilitate the expression of CSC markers, and caspases-4 and -5, on neoplastic epithelial cells. Expression of caspases-4 and -5 within epithelial cells may have a different role than within infiltrating immune cells of the stroma. Previous analysis of the role of NFKAPPAB in both epithelial and infiltrating myeloid cells during a murine model of colitis-associated CRC revealed that activation of this inflammatory transcription factor in the two different cell types drives carcinogenesis through distinct mechanisms, involving stimulation of pro-inflammatory cytokines by infiltrating myeloid cells; and prevention of death of IECs with tumorigenic potential_ENREF_32. Caspase-4 has previously been attributed with a role in LPS-mediated driving NFKAPPAB activation_ENREF_33. Thus, caspases-4 and -5 may be responsible for driving tumor initiation by preventing the death of IECs during CRC.

We present the expression of caspase-4 and -5 within intestinal epithelial cells as novel and highly specific biomarkers of colorectal carcinoma. Our findings may be relevant for the diagnosis of CRC, as diagnostic tests to assess caspase-4/-5 expression levels within IECs may be developed in light of these observations. Furthermore, our findings that caspase-4 and -5 contribute to the inflammatory status in IBD patients reveals these two caspases as novel targets for dampening intestinal inflammation.

### EXAMPLE 2A - OESOPHAGEAL INFLAMMATION (BARRETTS) AND OESOPHAGEAL ADENOCARCINOMA (OAC)

Having observed striking differences in cellular expression levels of caspases-4 and -5 in normal, inflamed and malignant colon tissue, we sought to determine whether similar differences were also apparent in other tissues within the and GI tract.

### Methods

### Clinical samples- Oesophageal tissue

Ethical approval was obtained from the St. James Hospital/TCD Ethics committee. Resection tissue from five Barretts/OAC patients were assessed by a clinical pathologist and defined areas of adjacent normal, metaplastic and tumour regions were identified. IHC staining for caspase-4 and -5 were carried out on each defined region using a BOND-III immunostainer from Leica Biosystems, Newcastle, UK. The Bond-III system dewaxed slides before pre-treatment with BOND Epitope Retrieval Solution I (Cat. no. AR9961). IHC scoring was performed (blinded) by multiplying the percentage of positive cells (P) by the intensity (I) of cytoplasmic staining. Percentage of positive cells was assessed using 6 categories (0%, 10%, 25%, 50%, 75%, 90% and 100%). Intensity of staining was graded using a scale of 0 - 3 where 0 = negative, 1 = weak, 2 = moderate and 3 = strong staining.

### Results

Inflammatory diseases in tissues of the and GI tract, such as the throat, oesophagus, stomach and pancreas are associated with the development of adenocarcinoma. Tissue resections from five OAC patients were H&E stained, and defined regions of normal, inflamed and tumour tissue were identified by an experienced clinical pathologist. 3 cores from each of the normal, inflamed and tumour areas were taken from each patient resection and a tissue microarray (TMA) slide was generated. TMA slides were IHC stained for caspases-4 and -5 and blindly scored for intensity and percentage positivity (Figure 12). Scoring and representative images of caspase-4 stained tissues (Fig. 12A-C) show low expression of caspase-4 in normal tissue, and increased expression of caspase-4 in both epithelial and stromal layers in metaplastic and tumour tissues. Caspase-5 staining showed a very similar expression pattern, with protein levels increasing from background tumour tissue of both stromal and epithelial cell layers (Fig. 12D-F).

### Discussion

These results identify caspases-4 and -5 as novel markers of oesophageal inflammation and OAC. Although we did not observe the specific expression of caspases-4 and -5 in malignant oesophageal tissue only, we postulate this is due to the fact that there are two epithelial cells types present in the oesophageal tissue: squamous and columnar epithelial cells. The scoring system used to generate the data presented here included both epithelial cell types, as it is difficult to distinguish the two cell types, particularly to the untrained eye. We postulate that, similar to our observations in colon tissue, adenomatous epithelial cells may remain negative for caspase-4/caspase-5 expression in inflamed/metaplastic tissue, and only become positively stained in malignant tissue.

### EXAMPLE 2B - OESOPHAGEAL INFLAMMATION (BARRETTS) AND OESOPHAGEAL ADENOCARCINOMA (OAC)

### Methods

### Clinical samples- Oesophageal tissue

Oesophagectomy tissues were retrieved from 32 oesophageal adenocarcinoma (OAC) patients and tissue microarrays (TMA) were constructed from formalin-fixed paraffin-embedded pre-treatment tumour biopsies (see Table 3 for patient characteristics). To account for heterogeneity, three cores were sampled from different areas of adjacent normal, Barrett's oesophagus (metaplasia) and oesophageal adenocarcinoma (tumour) patient resection tissues. Defined areas of tumour grades were assigned by a pathologist with TNM (Tumour-Node-Metastasis) clinical staging classification scale. 13 patients in this cohort received a course of neoadjuvant therapy (NAT) according to best clinical practice for treatment of their disease.

IHC staining for caspase-4 and -5 were carried out on each defined region using a BOND-III immunostainer from Leica Biosystems, Newcastle, UK. The Bond-III system dewaxed slides before pre-treatment with BOND Epitope Retrieval Solution I (Cat. no. AR9961).. IHC scoring was performed (blinded) by multiplying the percentage of positive cells (P) by the intensity (I) of cytoplasmic staining. Percentage of positive cells was assessed using 6 categories (0%, 10%, 25%, 50%, 75%, 90% and 100%). Intensity of staining was graded using a scale of 0 - 3 where 0 = negative, 1 = weak, 2 = moderate and 3 = strong staining.

**Table 3.**

| **Patient no.** | **NAT (Y/N)** | **Differentiation** | **Grade** | **Lymph node** |
|---|---|---|---|---|
| 1 | No | Poorly-differentiated adenocarcinoma of Gastrointestinal junction | Not stated | 2/8 nodes positive |
| 2 | Yes | Moderately differentiated adenocarcinoma | Not stated | Node negative |
| **3** | No | Adenocarcinoma, moderately differentiated | Not stated | 19 nodes negative |
| **4** | No | Adenocarcinoma poorly differentiated, diffuse, signet right type | Not stated | 11 nodes negative |
| **5** | Yes | Invasive moderately differentiated adenocarcinoma | ypT2N0Mx | Node negative |
| **7** | No | Invasive moderately differentiated adenocarcinoma | Not stated | 5/17 nodes positive |
| **9** | Yes | Poorly differentiated adenocarcinoma | yPT3N1Mx | 1 node positive |
| **10** | No | Intramucosal adenocarcinoma, well differentiated | pT1N0Mx | Node negative |
| **11** | Yes | Extensive residual poorly differentiated adenocarcinoma | ypT3, N1 | 3/10 positive |
| **12** | No | Moderately differentiated adenocarcinoma | pT3N1Mx | 4/6 positive |
| **14** | No | Invasive moderately differentiated adenocarcinoma | Not stated | 7 nodes negative |
| **15** | No | Adenocarcinoma, moderate to poorly differentiated | TIcN0MX | 25 nodes negative |
| **16** | No | Moderately differentiated adenocarcinoma | pT3N1Mx | 4/14 nodes positive |
| **17** | No | Ulcerated invasive poorly differentiated adenocarcinoma | pT3N1Mx | 4/10 nodes positive |
| **18** | No | Adenocarcinoma with no evidence of submucosal or deeper invasion | pT1N0Mx | 22 nodes negative |
| **19** | Yes | Residual invasive poorly differentiated adenocarcinoma with signet ring morphology and surface ulceration | ypT3N1Mx | 1/10 positive |
| **20** | Yes | Adenocarcinoma, differentiation: moderate | yPT2N0Mx | 40 node negative |
| **21** | No | Ulcerated invasive poorly differentiated adenocarcinoma | pT3N0(ITC)Mx | 19 nodes negative |
| **22** | Yes | Focal residual invasive adenocarcinoma | ypT2N0Mx | 5 nodes negative |
| **23** | No | Well differentiated adenocarcinoma with focal perineural invasion | pT3N0Mx | 10 nodes positive |
| **24** | No | Ulcerated invasive moderately and poorly differentiated adenocarcinoma | pT3pNlMx | 5/12 nodes positive |
| **25** | No | Ulcerated invasive poorly differentiated adenocarcinoma | pT2N0Mx | 14 nodes negative |
| **27** | Yes | Extensive, moderately differentiated adenocarcinoma | yPT3N1 | 1/8 nodes negative |
| **28** | No | Invasive moderate to focally poorly differentiated adenocarcinoma | ypT3N0Mx | 12 nodes negative |
| **29** | Yes | Moderately differentiated adenocarcinoma | ypT1N1Mx | 2/7 nodes positive |
| **30** | Yes | Invasive poorly differentiated adenocarcinoma | pT3N1Mx | 2/17 nodes positive |
| **31** | Yes | Invasive well to moderately differentiated adenocarcinoma | ypT3N1Mx | 3/18 nodes positive |
| **32** | Yes | Moderately to poorly differentiated adenocarcinoma | pT3N2Mx | 6/17 nodes positive |
| **34** | Yes | Poorly differentiated adenocarcinoma | ypT3N1Mx | 1/9 nodes positive |
| **35** | No | Moderate to well differentiated adenocarcinoma at oesophago-gastric junction | pT1 | Nodes negative |
| **38** | No | Invasive moderately differentiated oesophageal adenocarcinoma | pT1bN0Mx | Nodes negative |
| **39** | No | Intramucosal adenocarcinoma, moderately differentiated | pT1aN0Mx | 17 nodes negative |

### Results

The results are shown in figures 18.

Subsequent analysis of all three inflammatory caspases in a larger patient cohort (n=32) confirmed the previous observation that epithelial caspase-5 expression is low in normal tissue, and increases significantly during inflammation (metaplasia) and malignancy (Figure 18I, J). Although not as significant, caspase-4 expression is also increased during metaplasia (Figure 18F, H). Caspase-1 was expressed at low levels in non-inflamed tissue and at significantly increased levels in metaplastic and tumour tissues (in both stromal and epithelial cells), suggesting its involvement in BO and OAC associated inflammation (Figure 18A-D).Therefore, caspases-1 and -5 expression levels appear to correlate with inflammation/metaplasia during Barrett's Oesophagus, and epithelial caspase-5 expression levels are the only ones which are significantly elevated during OAC malignancy compared to metaplasia (Figure 18J).

### Discussion of Examples 2A and 2B

These results identify caspases-1, - and -5 as novel markers of oesophageal inflammation; and caspase-5 as a marker for OAC. Although we did not observe expression of caspase-5 exclusively in malignant epithelial oesophageal tissue, its expression levels were significantly increased during both metaplasia and malignancy, suggesting that it may serve as a novel biomarker for inflamed and malignant oesophageal tissue.

Furthermore, it is worth considering that there are two epithelial cells types present in the oesophageal tissue: squamous and columnar epithelial cells. The scoring system used to generate the data presented here included both epithelial cell types, as it is difficult to distinguish the two cell types, particularly to the untrained eye. We postulate that, similar to our observations in colon tissue, adenomatous epithelial cells may remain negative for caspase-5 expression in inflamed/metaplastic tissue, and only become positively stained in malignant tissue.

### EXAMPLE 3 - BREAST ADENOCARCINOMA

### Methods

### Clinical samples- Breast tissue

Ethical approval was obtained from the Beaumont Hospital Ethics committee. Tissue microarray (TMA) slides that had been previously constructed as validation TMAs, so all cores were aimed at tumour areas of breast adenocarcinoma tissue. IHC staining for caspase-4 and -5 were carried out on TMAs using a BOND-III immunostainer from Leica Biosystems, Newcastle, UK. The Bond-III system dewaxed slides before pre-treatment with BOND Epitope Retrieval Solution I (Cat. no. AR9961). IHC images were analysed in collaboration with the Pathology dept., Beaumont hospital.

### Results

To explore the hypothesis that caspases-4 and -5 may be general markers of adenocarcinoma, and that their specific expression in malignant adenomatous epithelial cells may not be limited to the colon, or to the and GI tract, we IHC stained a series of breast adenocarcinoma TMAs for caspase-4 and -5 expression. The staining patterns were carefully examined with a clinical pathologist. Representative images shown in Figure 13 highlight the observations that, although lymphocyte/stromal staining is positive for caspase-4 in all tissue areas, epithelial positivity appears to be restricted to tumour areas. Similar staining patterns were observed with caspase-5 staining also (data not shown). This data suggests that epithelial caspase-4 and -5 expression may be a specific marker of breast adenocarcinoma. In light of these recent findings, we postulate that caspases-4 and -5 may exist as general markers of adenocarcinoma in other tissues, such as additional areas within the and GI tract, larynx, prostate, vagina and lung etc.

### REFERENCES

1. Kamada S, Funahashi Y and Tsujimoto Y. (1997) Caspase-4 and caspase-5, members of the ICE/CED-3 family of cysteine proteases, are CrmA-inhibitable proteases. Cell Death Differ. 4; 473-8.
2. Mahida YR, Wu K, Jewell DP. Enhanced production of interleukin 1-beta by mononuclear cells isolated from mucosa with active ulcerative colitis of Crohn's disease. Gut 1989;30(6):835-8.
3. Pizarro TT, Michie MH, Bentz M, et al. IL-18, a novel immunoregulatory cytokine, is upregulated in Crohn's disease: expression and localization in intestinal mucosal cells. J Immunol 1999;162(11):6829-35.
4. Apte RN, Krelin Y, Song X, et al. Effects of micro-environment- and malignant cell-derived interleukin-1 in carcinogenesis, tumour invasiveness and tumour-host interactions. European journal of cancer (Oxford, England: 1990) 2006;42(6):751-9.
5. Yaguchi T, Saito M, Yasuda Y and Nishizaki T. (2010) Caspase-4 activation in association with decreased adenosine deaminase activity may be a factor for gastric ulcer. Digestion. 81:62-7. doi: 10.1159/000252771.
6. Soung YH, Jeong EG, Ahn CH, et al. Mutational analysis of caspase 1, 4, and 5 genes in common human cancers. Human pathology 2008;39(6):895-900.
7. Schwartz S, Jr., Yamamoto H, Navarro M, Maestro M, Reventos J, Perucho M. Frameshift mutations at mononucleotide repeats in caspase-5 and other target genes in endometrial and gastrointestinal cancer of the microsatellite mutator phenotype. Cancer Res 1999; 59(12):2995-3002.
8. Kayagaki N, Warming S, Lamkanfi M, et al. Non-canonical inflammasome activation targets caspase-11. Nature 2011;479(7371):117-21.
9. Demon D, Kuchmiy A, Fossoul A, Zhu Q, Kanneganti TD and Lamkanfi M. (2014) Caspase-11 is expressed in the colonic mucosa and protects against dextran sodium sulfate-induced colitis. Mucosal Immunol. 2014 May 21. doi: 10.1038/mi.2014.36. [Epub ahead of print]
10. Jarry A, Vallette G, Cassagnau E, et al. Interleukin 1 and interleukin 1beta converting enzyme (caspase 1) expression in the human colonic epithelial barrier. Caspase 1 downregulation in colon cancer. Gut 1999;45(2):246-51.
11. Ullman T, Odze R, Farraye FA. Diagnosis and management of dysplasia in patients with ulcerative colitis and Crohn's disease of the colon. Inflamm Bowel Dis 2009;15(4):630-8.
12. Sollberger G, Strittmatter GE, Kistowska M, French LE, Beer HD. Caspase-4 is required for activation of inflammasomes. J Immunol 2012;188(4):1992-2000.

## Claims

1. An in vitro method for the diagnosis and/or detection of and GI tract carcinoma, comprising the steps of
(a) monitoring and/or detecting the level of expression of caspase-4 and/or caspase-5 in a and GI tract epithelial tissue and/or cells from a subject sample or biopsy;
(b) comparing the level of expression of caspase-4 and/or caspase-5 in the sample or biopsy to the level of expression of caspase-4 and/or caspase-5 from a normal (negative) control and/or a positive control; and
(c) identifying and/or diagnosing and GI tract carcinoma when a detectable level or positive deviation of the expression levels of caspase-4 and/or caspase-5 compared to a normal (negative) control and/or a positive control is present within the and GI tract epithelial tissue and/or cells sample or biopsy.

2. The method according to claim 1, further comprising the steps of
(a) monitoring and/or detecting the level of expression of caspase-1 in epithelial tissue and/or cells from a subject sample or biopsy;
(b) comparing the level of expression of caspase-1 in the sample or biopsy to the level of expression of caspase-1 from a normal (negative) control and/or a positive control; and
(c) identifying and/or diagnosing malignancy when a negative deviation of the expression levels of caspase-1 compared to a normal (negative) control and/or a positive control is present within the epithelial tissue and/or cells sample or biopsy.

3. The method according to any of the preceding claims for early diagnosis and/or detection of carcinoma, wherein increased caspase-5 expression levels provide for early diagnosis and/or detection of carcinoma.

4. The method according to any of the preceding claims wherein the carcinoma is selected from an adenocarcinoma, squamous cell carcinoma, basal cell carcinoma or transitional cell carcinoma.

5. The method according to any of the preceding claims, wherein the GI tract carcinoma is selected from cancer of the oesophagus, stomach, biliary system, pancreas, small intestine, large intestine, rectum and anus.

6. The method according to any of the preceding claims for the identification of inflamed or malignant intestinal tissue and/or associated disease state comprising the steps of
(a) monitoring and/or detecting the level of expression of caspase-4 and/or caspase-5 in an intestinal stromal and/or epithelial tissue sample or a stool sample comprising stromal and/or epithelial intestinal cells from a subject;
(b) comparing the level of expression of caspase-4 and/or caspase-5 in the sample to the level of expression of caspase-4 and/or caspase-5 from a normal (negative) control and/or a positive control;
(c) identifying inflamed intestinal tissue and/or associated disease state when a detectable level or positive deviation of the expression levels of caspase-4 and/or caspase-5 compared to a normal (negative) control and/or a positive control is present within the intestinal stromal tissue sample or a stool sample; and/or
(d) identifying malignant intestinal tissue and/or associated disease state, including early stage adenoma and/or colorectal cancer (CRC), when a detectable level or positive deviation of the expression levels of caspase-4 and/or caspase-5 compared to a normal (negative) control and/or a positive control is present within the intestinal epithelial tissue sample or a stool sample.

7. The method according to claim 6 wherein the associated disease state of step (c) is selected from inflammatory bowel disease (IBD), Crohn's disease (CD), ulcerative colitis (UC).

8. The method according to claim 6 for the early identification and diagnosis of IBD, including Crohn's disease (CD) and/or ulcerative colitis (UC), wherein detectable, increased or upregulated expression of caspase-4 and/or caspase-5 in non-inflamed stromal cells from the subject sample, compared to the level of expression of caspase-4 and/or caspase-5 in stromal cells from a normal control, is indicative of IBD, including CD and/or UC.

9. The method according to claim 6 for the identification and early diagnosis of CRC wherein detectable, increased or upregulated expression of caspase-4 and/or caspase-5 levels in epithelial cells from the subject sample, compared to the level of expression of caspase-4 and/or caspase-5 in epithelial cells from a normal control, is indicative of early stage colorectal cancer (CRC).

10. The method according to any of claims 6-9, further comprising monitoring and/or detecting the level of expression of caspase-1 in an intestinal epithelial tissue sample or a stool sample comprising epithelial intestinal cells from a subject; comparing the level of expression of caspase-1 in the sample to the level of expression of caspase-1 from a normal (negative) control and/or a positive control; and identifying and/or diagnosing malignancy when a negative deviation of the expression levels of caspase-1 compared to a normal (negative) control and/or a positive control is present within the tissue and/or cells sample or biopsy.

11. Caspase-4 and/or caspase-5 inhibitors for use in the treatment of carcinoma, wherein the carcinoma is a GI tract cancer optionally selected from cancers of the oesophagus, stomach, biliary system, pancreas, small intestine, large intestine, rectum and anus, colorectal cancer (CRC) or early stage adenoma in a subject.

12. Caspase-4 and/or caspase-5 inhibitors for use according to claim 11 in the treatment of early stage carcinoma.

13. A caspase-1 modulator to increase caspase-1 expression levels or recombinant caspase-1 for use in the treatment of carcinoma wherein the carcinoma is a GI tract cancer optionally selected from cancers of the oesophagus, stomach, biliary system, pancreas, small intestine, large intestine, rectum and anus, colorectal cancer (CRC) or early stage adenoma in a subject.

14. A caspase-1 modulator for use according to claim 13 in the treatment of early stage carcinoma.

## Patentansprüche

1. In-vitro-Verfahren zur Diagnose und/oder Detektion eines Karzinoms des Gastrointestinaltrakts, wobei das Verfahren die folgenden Schritte umfasst:
(a) Überwachen und/oder Detektieren des Expressionsgrads von Caspase-4 und/oder Caspase-5 in Epithelgewebe und/oder -zellen des Gastrointestinaltrakts von einer Probe oder Biopsie eines Subjekts;
(b) Vergleichen des Expressionsgrads von Caspase-4 und/oder Caspase-5 in der Probe oder Biopsie mit dem Expressionsgrad von Caspase-4 und/oder Caspase-5 von einer normalen (negativen) Kontrolle und/oder einer positiven Kontrolle; und
(c) Identifizieren und/oder Diagnostizieren eines Karzinoms des Gastrointestinaltrakts, wenn ein detektierbarer Grad oder eine positive Abweichung der Expressionsgrade von Caspase-4 und/oder Caspase-5 verglichen mit einer normalen (negativen) Kontrolle und/oder einer positiven Kontrolle in der Epithelgewebe- und/oder Epithelzellprobe oder -biopsie des Gastrointestinaltrakts vorhanden ist.

2. Verfahren nach Anspruch 1, ferner die folgenden Schritte umfassend:
(a) Überwachen und/oder Detektieren des Expressionsgrads von Caspase-1 in Epithelgewebe und/oder -zellen von einer Probe oder Biopsie eines Subjekts;
(b) Vergleichen des Expressionsgrads von Caspase-1 in der Probe oder Biopsie mit dem Expressionsgrad von Caspase-1 von einer normalen (negativen) Kontrolle und/oder einer positiven Kontrolle; und
(c) Identifizieren und/oder Diagnostizieren einer Malignität, wenn eine negative Abweichung der Expressionsgrade von Caspase-1 verglichen mit einer normalen (negativen) Kontrolle und/oder einer positiven Kontrolle in der Epithelgewebe- und/oder Epithelzellprobe oder -biopsie vorhanden ist.

3. Verfahren nach einem der vorstehenden Ansprüche zur frühzeitigen Diagnose und/oder Detektion eines Karzinoms, wobei erhöhte Expressionsgrade von Caspase-5 einer frühzeitigen Diagnose und/oder Detektion eines Karzinoms dienen.

4. Verfahren nach einem der vorstehenden Ansprüche, wobei das Karzinom ausgewählt ist aus einem Adenokarzinom, einem squamösen Zellkarzinom, einem Basalzellkarzinom oder einem Übergangszellkarzinom.

5. Verfahren nach einem der vorstehenden Ansprüche, wobei das Karzinom des Gastrointestinaltrakts ausgewählt ist aus einem Krebs der Speiseröhre, des Magens, des Gallengangsystems, der Bauchspeicheldrüse, des Dünndarms, des Dickdarms, des Rektums und des Anus.

6. Verfahren nach einem der vorstehenden Ansprüche zur Identifikation von entzündetem oder malignem Intestinalgewebe und/oder einem verbundenen Erkrankungszustand, wobei das Verfahren die folgenden Schritte umfasst:
(a) Überwachen und/oder Detektieren des Expressionsgrads von Caspase-4 und/oder Caspase-5 in einer Intestinalstroma- und/oder Intestinalepithelgewebeprobe oder einer Stuhlprobe umfassend Stroma- und/oder Epithelintestinalzellen von einem Subjekt;
(b) Vergleichen des Expressionsgrads von Caspase-4 und/oder Caspase-5 in der Probe mit dem Expressionsgrad von Caspase-4 und/oder Caspase-5 von einer normalen (negativen) Kontrolle und/oder einer positiven Kontrolle;
(c) Identifizieren von entzündetem Intestinalgewebe und/oder einem verbundenen Erkrankungszustand, wenn ein detektierbarer Grad oder eine positive Abweichung der Expressionsgrade von Caspase-4 und/oder Caspase-5 verglichen mit einer normalen (negativen) Kontrolle und/oder einer positiven Kontrolle in der Intestinalstromagewebeprobe oder einer Stuhlprobe vorhanden ist; und/oder
(d) Identifizieren von malignem Intestinalgewebe und/oder einem verbundenen Erkrankungszustand, einschließlich eines Adenoms und/oder Dickdarmkrebs (colorectal cancer - CRC) im Frühstadium, wenn ein detektierbarer Grad oder eine positive Abweichung der Expressionsgrade von Caspase-4 und/oder Caspase-5 verglichen mit einer normalen (negativen) Kontrolle und/oder einer positiven Kontrolle in der Intestinalepithelgewebeprobe oder einer Stuhlprobe vorhanden ist.

7. Verfahren nach Anspruch 6, wobei der verbundene Erkrankungszustand aus Schritt (c) ausgewählt ist aus einer entzündlichen Darmerkrankung (inflammatory bowel disease - IBD), Morbus Crohn (Crohn's disease - CD) oder Colitis ulcerosa (ulcerative colitis - UC).

8. Verfahren nach Anspruch 6 zur frühzeitigen Identifikation und Diagnose von IBD, einschließlich Morbus Crohn (CD) und/oder Colitis ulcerosa (UC), wobei eine detektierbare, erhöhte oder hochregulierte Expression von Caspase-4 und/oder Caspase-5 in nichtentzündeten Stromazellen von der Probe des Subjekts, verglichen mit dem Expressionsgrad von Caspase-4 und/oder Caspase-5 in Stromazellen von einer normalen Kontrolle, auf IBD, einschließlich CD und/oder UC, hindeutet.

9. Verfahren nach Anspruch 6 zur Identifikation und frühzeitigen Diagnose von CRC, wobei eine detektierbare, erhöhte oder hochregulierte Expression von Caspase-4- und/oder Caspase-5-Graden in Epithelzellen von der Probe des Subjekts, verglichen mit dem Expressionsgrad von Caspase-4 und/oder Caspase-5 in Epithelzellen von einer normalen Kontrolle, auf Dickdarmkrebs (CRC) im Frühstadium hindeutet.

10. Verfahren nach einem der Ansprüche 6-9, ferner umfassend ein Überwachen und/oder Detektieren des Expressionsgrads von Caspase-1 in einer Intestinalepithelgewebeprobe oder einer Stuhlprobe, umfassend Epithelintestinalzellen von einem Subjekt; ein Vergleichen des Expressionsgrads von Caspase-1 in der Probe mit dem Expressionsgrad von Caspase-1 von einer normalen (negativen) Kontrolle und/oder einer positiven Kontrolle; und Identifizieren und/oder Diagnostizieren einer Malignität, wenn eine negative Abweichung der Expressionsgrade von Caspase-1 verglichen mit einer normalen (negativen) Kontrolle und/oder einer positiven Kontrolle in der Gewebe- und/oder Zellprobe oder -biopsie vorhanden ist.

11. Caspase-4- und/oder Caspase-5-Inhibitoren zur Verwendung bei der Behandlung eines Karzinoms, wobei das Karzinom ein Krebs des Gastrointestinaltrakts ist, optional ausgewählt aus Krebserkrankungen der Speiseröhre, des Magens, des Gallengangsystems, der Bauchspeicheldrüse, des Dünndarms, des Dickdarms, des Rektums und des Anus, Dickdarmkrebs (CRC) oder einem Adenom im Frühstadium in einem Subjekt.

12. Caspase-4- und/oder Caspase-5-Inhibitoren zur Verwendung nach Anspruch 11 bei der Behandlung eines Karzinoms im Frühstadium.

13. Caspase-1-Modulator zum Erhöhen von Expressionsgraden von Caspase-1 oder rekombinantem Caspase-1 zur Verwendung bei der Behandlung eines Karzinoms, wobei das Karzinom ein Krebs des Gastrointestinaltrakts ist, optional ausgewählt aus Krebserkrankungen der Speiseröhre, des Magens, des Gallengangsystems, der Bauchspeicheldrüse, des Dünndarms, des Dickdarms, des Rektums und des Anus, Dickdarmkrebs (CRC) oder einem Adenom im Frühstadium in einem Subjekt.

14. Caspase-1-Modulator zur Verwendung nach Anspruch 13 bei der Behandlung eines Karzinoms im Frühstadium.

## Revendications

1. Procédé in vitro pour le diagnostic et/ou la détection d'un carcinome du tractus GI, comprenant les étapes
(a) de surveillance et/ou de détection du niveau d'expression de la caspase-4 et/ou de la caspase-5 dans un tissu épithélial et/ou des cellules épithéliales du tractus GI à partir d'un échantillon ou d'une biopsie d'un sujet ;
(b) de comparaison du niveau d'expression de la caspase-4 et/ou de la caspase-5 dans l'échantillon ou la biopsie au niveau d'expression de la caspase-4 et/ou de la caspase-5 à partir d'un contrôle normal (négatif) et/ou d'un contrôle positif ; et
(c) d'identification et/ou de diagnostic d'un carcinome du tractus GI lorsqu'un niveau détectable ou un écart positif des niveaux d'expression de la caspase-4 et/ou de la caspase-5 par rapport à un contrôle normal (négatif) et/ou un contrôle positif est présent dans l'échantillon ou la biopsie de tissus épithéliaux et/ou cellules épithéliales du tractus GI.

2. Procédé selon la revendication 1, comprenant en outre les étapes
(a) de surveillance et/ou de détection du niveau d'expression de la caspase-1 dans les tissus épithéliaux et/ou les cellules épithéliales à partir d'un échantillon ou d'une biopsie d'un sujet ;
(b) de comparaison du niveau d'expression de la caspase-1 dans l'échantillon ou la biopsie au niveau d'expression de la caspase-1 à partir d'un contrôle normal (négatif) et/ou d'un contrôle positif ; et
(c) d'identification et/ou de diagnostic d'une tumeur maligne lorsqu'un écart négatif des niveaux d'expression de la caspase-1 par rapport à un contrôle normal (négatif) et/ou un contrôle positif est présent dans l'échantillon ou la biopsie de tissus épithéliaux et/ou cellules épithéliales.

3. Procédé selon l'une quelconque des revendications précédentes pour un diagnostic et/ou une détection précoces du carcinome, dans lequel des niveaux d'expression accrus de caspase-5 permettent un diagnostic et/ou une détection précoces du carcinome.

4. Procédé selon l'une quelconque des revendications précédentes, dans lequel le carcinome est choisi parmi un adénocarcinome, un carcinome épidermoïde, un carcinome basocellulaire ou un carcinome à cellules transitionnelles.

5. Procédé selon l'une quelconque des revendications précédentes, dans lequel le carcinome du tractus GI est choisi parmi le cancer de l'œsophage, de l'estomac, du système biliaire, du pancréas, de l'intestin grêle, du gros intestin, du rectum et de l'anus.

6. Procédé selon l'une quelconque des revendications précédentes pour l'identification d'un tissu intestinal enflammé ou malin et/ou d'un état pathologique associé comprenant les étapes
(a) de surveillance et/ou de détection du niveau d'expression de la caspase-4 et/ou de la caspase-5 dans un échantillon de tissus stromaux et/ou épithéliaux intestinaux ou un échantillon de selles comprenant des cellules intestinales stromales et/ou épithéliales d'un sujet ;
(b) de comparaison du niveau d'expression de caspase-4 et/ou caspase-5 dans l'échantillon au niveau d'expression de caspase-4 et/ou caspase-5 à partir d'un contrôle normal (négatif) et/ou d'un contrôle positif ;
(c) d'identification de tissus intestinaux enflammés et/ou de l'état pathologique associé lorsqu'un niveau détectable ou un écart positif des niveaux d'expression de caspase-4 et/ou caspase-5 par rapport à un contrôle normal (négatif) et/ou un contrôle positif est présent dans un échantillon de tissus stromaux intestinaux ou un échantillon de selles ; et/ou
(d) d'identification de tissus intestinaux malins et/ou d'un état pathologique associé, y compris l'adénome de stade précoce et/ou le cancer colorectal (CRC), lorsqu'un niveau détectable ou un écart positif des niveaux d'expression de caspase-4 et/ou caspase-5 par rapport à un contrôle normal (négatif) et/ou un contrôle positif est présent dans l'échantillon de tissus épithéliaux intestinaux ou un échantillon de selles.

7. Procédé selon la revendication 6, dans lequel l'état pathologique associé de l'étape (c) est choisi parmi la maladie inflammatoire de l'intestin (MII), la maladie de Crohn (MC), la colite ulcéreuse (CU).

8. Procédé selon la revendication 6 pour l'identification et le diagnostic précoces de la MII, y compris la maladie de Crohn (MC) et/ou la colite ulcéreuse (CU), dans lequel l'expression détectable, augmentée ou régulée à la hausse de la caspase-4 et/ou de la caspase-5 dans des cellules stromales non enflammées de l'échantillon d'un sujet, par rapport au niveau d'expression de la caspase-4 et/ou de la caspase-5 dans les cellules stromales à partir d'un contrôle normal, indique une MII, y compris une MC et/ou une CU.

9. Procédé selon la revendication 6 pour l'identification et le diagnostic précoce de CRC dans lequel l'expression détectable, augmentée ou régulée à la hausse des niveaux d'expression de caspase-4 et/ou caspase-5 dans des cellules épithéliales de l'échantillon d'un sujet, par rapport au niveau d'expression de la caspase-4 et/ou de la caspase-5 dans les cellules épithéliales à partir d'un contrôle normal, indique un cancer colorectal de stade précoce (CRC).

10. Procédé selon l'une quelconque des revendications 6 à 9, comprenant en outre la surveillance et/ou la détection du niveau d'expression de la caspase-1 dans un échantillon de tissus épithéliaux intestinaux ou un échantillon de selles comprenant des cellules intestinales épithéliales d'un sujet ; la comparaison du niveau d'expression de la caspase-1 dans l'échantillon au niveau d'expression de la caspase-1 à partir d'un contrôle normal (négatif) et/ou d'un contrôle positif ; et l'identification et/ou le diagnostic d'une tumeur maligne lorsqu'un écart négatif des niveaux d'expression de la caspase-1 par rapport à un contrôle normal (négatif) et/ou un contrôle positif est présent dans l'échantillon ou la biopsie de tissus et/ou cellules.

11. Inhibiteurs de caspase-4 et/ou de caspase-5 destinés à être utilisés dans le traitement du carcinome, dans lesquels le carcinome est un cancer du tractus GI éventuellement choisi parmi les cancers de l'œsophage, de l'estomac, du système biliaire, du pancréas, de l'intestin grêle, du gros intestin, du rectum et de l'anus, le cancer colorectal (CRC) ou l'adénome de stade précoce chez un sujet.

12. Inhibiteurs de caspase-4 et/ou caspase-5 destinés à être utilisés selon la revendication 11 dans le traitement du carcinome de stade précoce.

13. Modulateur de caspase-1 pour augmenter les niveaux d'expression de caspase-1 ou la caspase-1 recombinante destiné à être utilisé dans le traitement du carcinome dans lequel le carcinome est un cancer du tractus GI éventuellement choisi parmi les cancers de l'œsophage, de l'estomac, du système biliaire, du pancréas, de l'intestin grêle, du gros intestin, du rectum et de l'anus, le cancer colorectal (CRC) ou l'adénome de stade précoce chez un sujet.

14. Modulateur de caspase-1 destiné à être utilisé selon la revendication 13 dans le traitement du carcinome de stade précoce.
